# EUROPEAN PATENT SPECIFICATION

(11) **EP 1 034 273 B1**
(45) Date of publication and mention of the grant of the patent: **27.12.2006**
(21) Application number: 98955786.3
(22) Date of filing: 26.11.1998
(51) Int. Cl.: C12N 15/21, C07K 14/435, A61K 38/17, A61K 31/40, A61K 31/19, A61K 31/66, A61K 31/35, G01N 33/68, A23L 1/015, C12N 5/10, C12N 1/21, A01K 67/027, C12N 15/00

(54) **HISTAMINE AND SEROTONIN BINDING MOLECULES**
HISTAMINE UND SEROTONINE BINDENDE MOLEKÜLE
MOLECULES DE FIXATION DE L'HYSTAMINE ET DE LA SEROTONINE

(30) Priority: 26.11.1997 GB 9725046; 26.06.1998 GB 9813917
(43) Date of publication of application: 13.09.2000
(73) Proprietor: Evolutec Limited, Reading, Berkshire RG2 6UG (GB)
(72) Inventor: NUTTALL, Patricia, Ann, Culham Oxon OX14 4NP (GB); PAESEN, Guido, Christian, Oxford OX1 3TA (GB)
(74) Representative: Mercer, Christopher Paul
(86) International application number: PCT/GB1998/003530
(87) International publication number: WO 1999/027104

(56) References cited:
- WO-A-96/11271
- WO-A-97/44451
- RIBEIRO J M C ET AL: "HIGH AFFINITY HISTAMINE-BINDING AND ANTIHISTAMINIC ACTIVITY OF THE SALIVARY NITRIC OXIDE-CARRYING HEME PROTEIN (NITROPHORIN) OF RHODNIUS PROLIXUS" JOURNAL OF EXPERIMENTAL MEDICINE, vol. 180, December 1994, pages 2251-2257, XP002050815
- WANG H ET AL: "COMPARISON OF THE PROTEINS IN SALIVARY GLANDS, SALIVA AND HAEMOLYMPH OF RHIPICEPHALUS APPENDICULATUS FEMALE TICKS DURING FEEDING" PARASITOLOGY, vol. 109, no. 4, November 1994, pages 517-523, XP002050816
- PAESEN, G. C. (1) ET AL: "Histamine - binding proteins in tick saliva." FASEB JOURNAL, (MARCH 20, 1998) VOL. 12, NO. 5, PP. A1001. MEETING INFO.: ANNUAL MEETING OF THE PROFESSIONAL RESEARCH SCIENTISTS ON EXPERIMENTAL BIOLOGY 98, PART II SAN FRANCISCO, CALIFORNIA, USA APRIL 18-22, 1998 FEDERATION OF AMERICAN SOCIETIES FOR, XP002099797

## Description

The present invention relates to serotonin binding molecules. The molecules of the present invention may be used in the regulation of the action of serotonin and are thus useful in the detection and quantification of serotonin and in the treatment of various diseases and allergies.

Vasoactive amines such as histamine and serotonin are mediators of inflammation and regulators of certain physiological processes in animals, including humans. Histamine is present in the secretory granules of mast cells and basophils and is formed decarboxylation of histidine. It is also present in ergot and plants and may be synthesised synthetically from histidine or citric acid.

The main actions of histamine in humans are stimulation of gastric secretion, contraction of most smooth muscle tissue, cardiac stimulation, vasodilation and increased vascular permeability. In addition to its regulatory role in immune reactions and inflammatory processes, histamine also modulates the production of many cytokines in the body (including those that regulate inflammation) and can interfere with the expression of cytokine receptors. Furthermore, histamine promotes wound healing.

The main pathophysiological roles of histamine are as a stimulant of gastric acid secretion and as a mediator of type I hypersensitivity reactions such as urticaria and hay fever. Histamine and its receptors also have pathological aspects to their functions. They play dominant roles in allergies such as asthma, allergic rhinitis, atopic dermatitis and food and drug allergies, which affect a great number of people and are an important cause of illness and mortality. Histamine or its receptors may also be involved either directly or indirectly in autoimmune disease, e.g. arthritis, and in tumour growth (Falus, 1994).

The hormone serotonin (also known as 5-hydroxytryptamine) is both a vasoconstrictor and a neurotransmitter. It can also increase vascular permeability, induce dilation of capillaries and cause the contraction of nonvascular smooth muscle. Serotonin is present in the brain and intestinal tissues and is produced by the pineal gland and by blood platelets. Pathological aspects related to serotonin include abnormal blood pressure, migraine, psychological disorders, respiratory disease and coronary heart disease. Serotonin agonists and antagonists are used to treat some of these disorders, but again often have undesirable side-effects.

Anti-histamine drugs are widely used, especially for the treatment of allergies. Most of these drugs are compounds that are structurally related to histamine, and bind to its receptor(s), thereby obstructing the interaction of histamine with its receptor(s). Such drugs as are currently available often have undesirable side effects (for example drowsiness) and are not always effective.

Histamine produces its actions by an effect on specific histamine receptors which are of three main types, H₁, H₂ and H₃, distinguished by means of selective antagonist and agonist drugs. Histamine H₁ and H₂ antagonists have clinical uses but at present histamine H₃ antagonists are used mainly as research tools. Intracellular histamine appears to be involved in cellular growth (tumour growth promotion) and tissue repair. Currently undefined intracellular histamine receptors are thought to be involved in these processes (Falus, 1994).

Histamine receptors have been the subject of concentrated research for a number of years. However, scant information is available regarding the structure of the active site of these molecules - in fact the H₃ receptor has not yet been cloned. The lack of any direct structural information for these proteins is presumably due to the fact that histamine receptor proteins are membrane proteins that denature in the absence of lipid and are consequently very difficult to crystallise.

Based on the fact that the H₁ and H₂-type receptors belong to the broad class of seven transmembrane G protein-coupled receptors, it can be assumed that they are mainly alpha-helical. A number of site-directed mutagenesis studies have been performed on these receptors that have indicated certain residues that are important for histamine binding. In the H₂ receptor, Asp⁹⁸, Asp¹⁸⁶ and Thr¹⁹⁰ are believed to contribute to the histamine binding pocket (Gantz *et al.,* 1992).

Conventional H₁ receptor antagonists are widely used as antihistamines for treating allergic reactions including allergic rhinitis (hay fever), urticaria, insect bites and drug hypersensitivities. Drugs that lack sedative or muscarinic receptor antagonists are preferred. H₁ receptor antagonists are also used as anti-emetics for the prevention of motion sickness or other causes of nausea including severe morning sickness. Muscarinic receptor antagonist actions of some antihistamines probably contribute to efficacy but also cause side-effects. Some H₁ receptor antagonists are fairly strong sedatives and may be used for this action.

However, there are numerous undesirable effects of the H₁ receptor antagonists currently used. When used for purely antihistamine actions, all of the CNS effects are unwanted. When used for their sedative or anti-emetic actions, some of the CNS effects such as dizziness, tinnitus and fatigue are unwanted. Excessive doses can cause excitation and may produce convulsions in children. The peripheral anti-muscarinic actions are always undesirable. The commonest of these is dryness of the mouth, but blurred vision, constipation and retention of urine can also occur. Unwanted effects not related to the drug's pharmaceutical action are also seen. Thus, gastrointestinal disturbances are fairly common while allergic dermatitis can follow topical application of these drugs.

H₂ antagonists are frequently used as inhibitors of gastric acid secretion. They are used as the drugs of choice in the treatment of peptic ulcer, as second line drugs in the treatment of Zollinger-Ellison syndrome and for treating reflux oesophagitis. Unwanted effects have been reported that include diarrhoea, dizziness, muscle pains, transient rashes and hyper-gastrinaemia. Some H₂ receptor antagonists can cause gynaecomastia in men and confusion in the elderly.

Besides these unwanted effects, some histamine antagonists are troublesome if taken with alcohol or with drugs. For example, the antihistamine Seldane used in combination with antibiotics and antifungals may cause life-threatening side-effects.

It can therefore be seen that drugs used to control the actions of histamine are not always effective. The reasons why they may have limited efficacy may relate to the specificity of these drugs for only a sub-class of histamine receptors, particularly when a certain class of conditions require interference with a larger class of receptors. Molecules that actually bind to histamine itself would compete for histamine binding with all receptors and may thus be more suitable for treating certain conditions.

There is thus a great need for effective antagonists of histamine and serotonin that do not generate the side-effects that detract from their applicability to the treatment of human and animal disorders.

The use of molecules that bind to seratonin in its natural form that would replace antibodies in assays like these would provide a highly sensitive system for the measurement of unmodified seratonin.

Molecules capable of binding to histamine have previously been identified in blood-feeding ectoparasites. For example, a salivary nitric oxide-carrying haeme protein (nitrophorin) of the triatome bug *Rhodnius prolixus* has been found to bind histamine (Ribeiro and Walker, 1994). The isolation of four vasoactive amine binding proteins (VABPs) from ticks is described in co-pending International Patent Application No. PCT/GB97/01372 which is owned by the Applicant for the present invention. The contents of PCT/GB97/01372 are incorporated into the present application in their entirety.

These proteins bind to histamine and are closely related to one another. They are named MS-HBP1, FS-HBP1, FS-HBP2 and D.RET6. Some of these molecules also bind serotonin (for example FS-HBP2). In other cases, such as in the case of D.RET6 for example, binding of serotonin is thought to alter the affinity of the molecule for histamine. The DNA sequences that encode these proteins are presently being used to isolate other related proteins in the same family from the same and different species.

### Summary of the invention

According to a first aspect of the present invention there is provided the use of a serotonin binding compound capable of binding to serotonin with a dissociation constant of less than 10⁻⁷M and which has a binding site comprising amino acid residues phenylalanine, isoleucine or leucine at position I, tryptophan at position II and aspartate or glutamate at positions III and IV wherein residues I to IV are positioned substantially the same as residues 108, 42, 39 and 82 respectively in either of SEQ. ID. Nos 1 or 2, or residues 107, 41, 38 and 78 in SEQ ID 3 or residues 122, 54, 50 and 95 in SEQ ID 4, wherein the numbering of the amino acid residues refers to the sequence of the nature protein that locks the leader sequence, in the manufacture of a medicament for the treatment or prevention of a disease condition in which seratonin is impleatd, and wherein said disease conditions is abnormal blood pressure, migrane, a psycological disorder, respiratory disease or coronary heart disease. Hereafter, this binding site will be referred to as the "first binding site". The proteins identified in SEQ IDs I to 4 are known as FS-HBP1, FS-HBP2, MS-HBP and D.RET6 respectively.

According to a second aspect of the present invention there is provided a the use of a serotonin binding compound capable of binding to serotonin with a dissociation constant of less than 10⁻⁷M and which has a binding site comprising amino acid residues phenylalanine or isoleucine at residue I, tryptophan at residue II and aspartate or glutamate at residues III and IV wherein residues I to IV are positioned substantially the same as residues 98, 137, 24 and 120 respectively in either of SEQ. ID. Nos I or 2, or residues 95, 138,23 and 120 in SEQ. ID. 3 or residues 112, 149, 35 and 135 in SEQ. ID. 4, wherein the numbering of the amino acid residues refers to the sequence of the nature protein that locks the leader sequence, in the manufacture of a medicament for the treatment or prevention of a disease condition in which seratonin is impleatd, and wherein said disease conditions is abnormal blood pressure, migrane, a psycological disorder, respiratory disease or coronary heart disease. Hereafter, this binding site will be referred to as the "second binding site".

According to a third aspect of the invention there is provided a the use of a serotonin binding compound capable of binding to serotonin with a dissociation constant of less than 10⁻⁷M and which comprises both the first and second binding sites described above, wherein the numbering of the amino acid residues refers to the sequence of the nature protein that locks the leader sequence, in the manufacture of a medicament for the treatment or prevention of a disease condition in which seratonin is impleatd, and wherein said disease conditions is abnormal blood pressure, migrane, a psycological disorder, respiratory disease or coronary heart disease. For some histamine binding compounds containing both the first and second binding sites (such as D.RET6), binding of serotonin to the compound is thought to alter the affinity of the compound for histamine.

Other chemical compounds with a related action to serotonin may also influence the binding of histamine to a histamine binding compound containing both the first and the second binding sites. These related compounds include cysteinyl leukotrienes (such as leukotriene D₄ and leutkotriene E₄), platelet activating factor and thromboxanes.

According to a fourth aspect of the present invention there is provided a the use of a serotonin binding compound capable of binding to serotonin with a dissociation constant of less than 10⁻⁷M and which comprises the sequence of D.RET6 (SEQ. ID. 4) or a fragment thereof and which comprises a first and second binding site as defined above, wherein the numbering of the amino acid residues refers to the sequence of the nature protein that locks the leader sequence, in the manufacture of a medicament for the treatment or prevention of a disease condition in which seratonin is impleatd, and wherein said disease conditions is abnormal blood pressure, migrane, a psycological disorder, respiratory disease or coronary heart disease wherein binding of serotonin to the compound increases the affinity of the compound for histamine.

By binding site is meant the specific region in the compound that contributes directly to the binding of a histamine or serotonin molecule. As such, binding at this site will comprise molecular recognition events between the binding site and the histamine or serotonin molecule, regulated by functional complementarities of shape, size, charges, H-bonds, hydrophobic and pi interactions and van der Waal's forces. Interactions may also comprise covalent chemical bonds.

Current methods of generation of compounds with affinity for a molecule of interest have been until recently relatively primitive. The notion of combinatorial chemistry and the generation of combinatorial libraries has, however, developed at great speed and facilitated the rational design and improvement of molecules with desired properties. These techniques can be used to generate molecules possessing binding sites identical or similar to those of the histamine or serotonin binding sites identified herein.

Such compounds may be generated by rational design, using for example standard synthesis techniques in combination with molecular modelling and computer visualisation programs. Under these techniques, the "lead" compound with a similar framework to the histamine or serotonin binding site is optimised by combining a diversity of scaffolds and component substituents.

Alternatively, or as one step in the structure-guided design of a molecular entity, combinatorial chemistry may be used to generate or refine the structure of compounds that mimic the serotonin binding site of serotonin binding compounds by the production of congeneric combinatorial arrays around a framework scaffold. These steps might include standard peptide or organic molecule synthesis with a solid-phase split and recombine process or parallel combinatorial unit synthesis using either solid phase or solution techniques (see, for example Hogan, 1997 and the references cited therein).

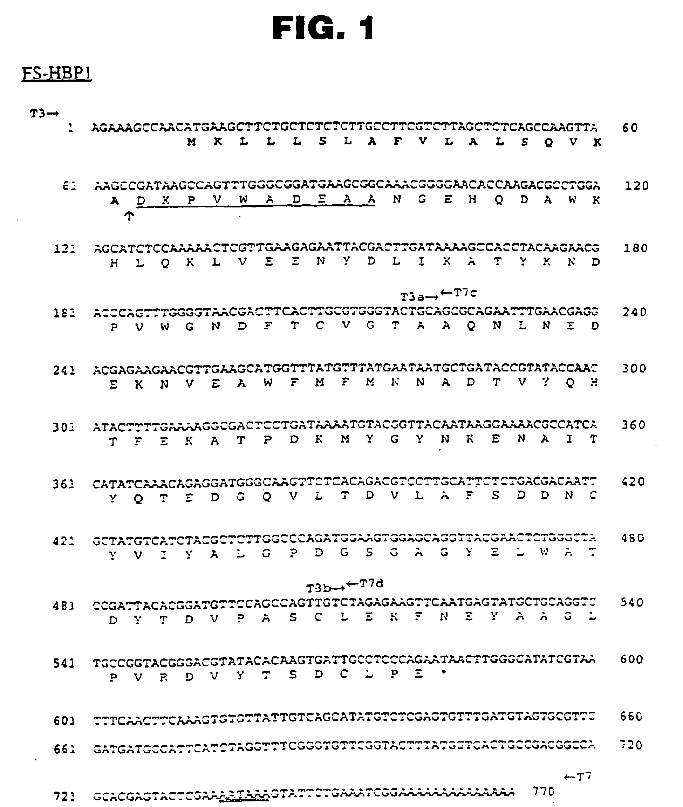

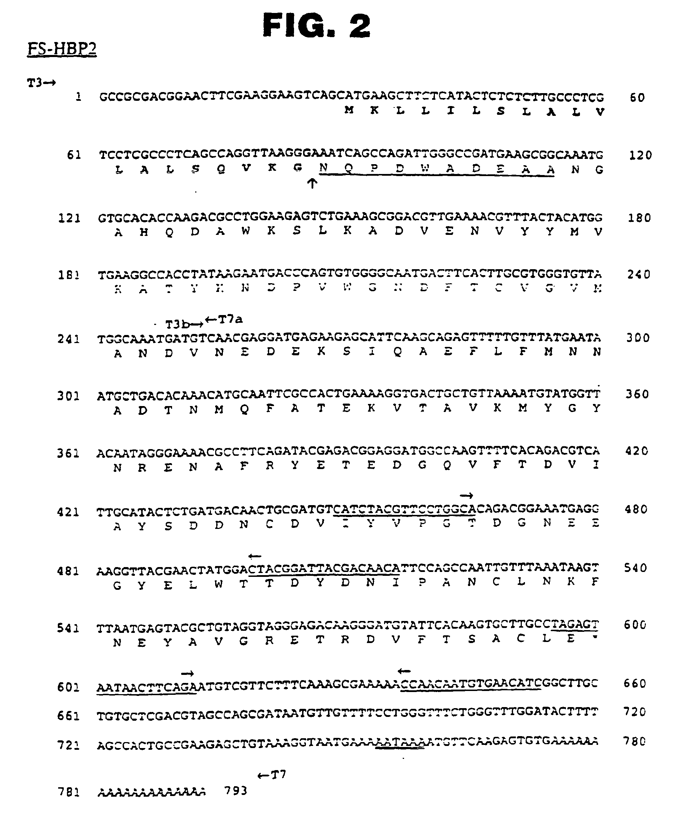

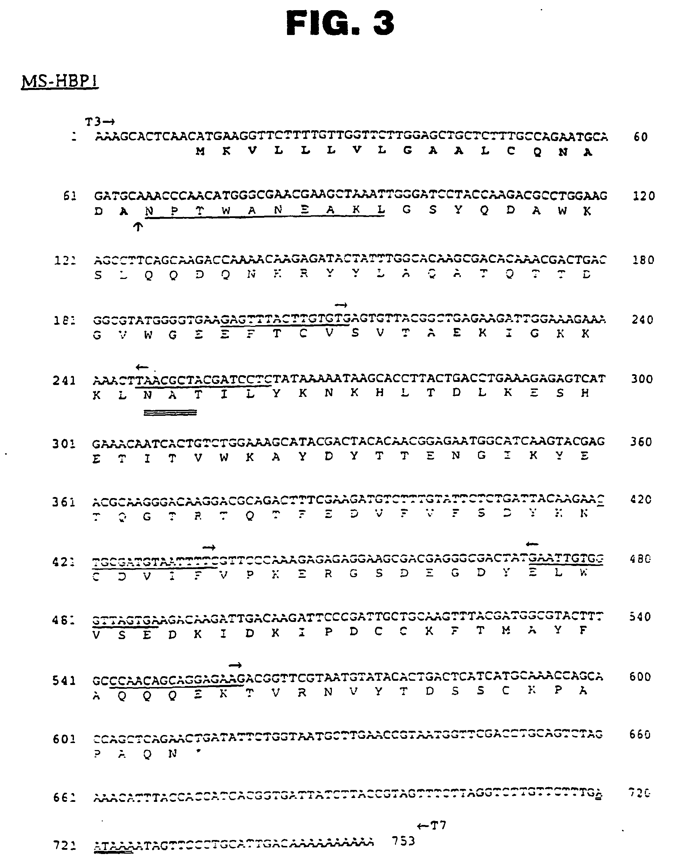

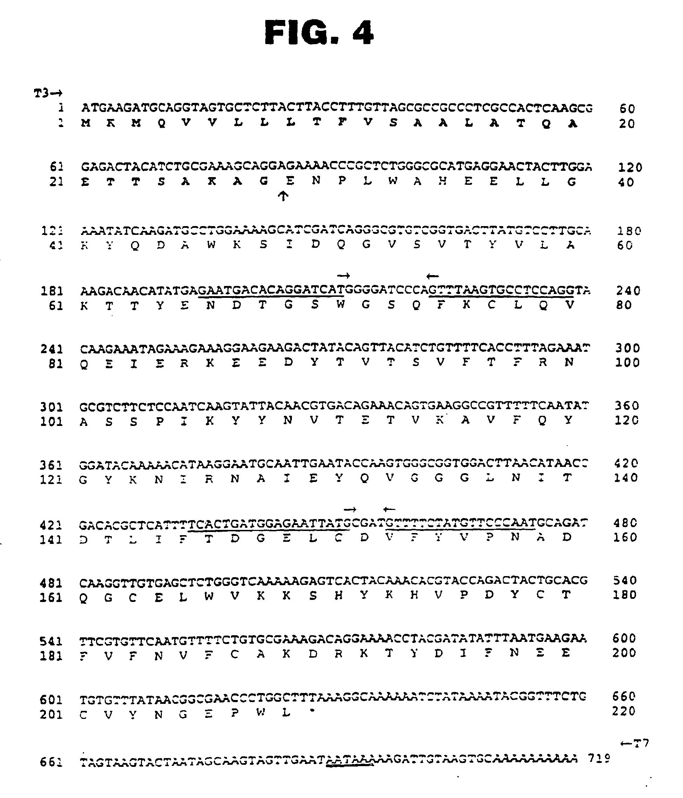

Alternatively, or as a portion of a serotonin binding molecule of the present invention, functional equivalents may comprise fragments or variants of the proteins identified in Figures 1 to 4 or closely related proteins exhibiting significant sequence homology. By fragments is meant any portion of the entire protein sequence that retains the ability to bind to vasoactive amines with a dissociation constant of 10⁻⁷M or less. Accordingly, fragments containing single or multiple amino acid deletions from either terminus of the protein or from internal stretches of the primary amino acid sequence form one aspect of the present invention. Variants may include, for example, mutants containing amino acid substitutions, insertions or deletions from the wild type sequence of Figures I to 4.

The man of skill in the art will understand that the residues that contribute to the binding of vasoactive amines in the four proteins explicitly identified herein are maintained in the relevant position for binding to histamine or serotonin through the framework structure of the protein. Thus, the framework residues of the proteins are responsible for the exact positioning of the binding amino acids.

Accordingly, it is contemplated that any molecular framework capable of retaining these amino acid side-chains in the necessary positions for binding to serotonin will be suitable for use in accordance with the present invention. Of particular suitability will be cyclic peptides held in a precise framework by their linking groups and bonds. The amino acid side chains may be held in a position substantially identical to their position in the histamine or serotonin binding site of native histamine or serotonin binding compounds. Preferably, the cyclic peptides comprise between 6 and 30 amino acids, preferably between 8 and 20 amino acids. Of particular suitability is the cyclic octapeptide Ala-Glu-Ala-Phe-Ala-Glu-Ala-Trp.

Biologically-active peptides with serotonin binding sites according to the present invention may be generated using phage libraries. Nucleic acids encoding amino acid residues identified as participants in the binding of histamine or serotonin, together with nucleic acid encoding the surrounding framework residues may be fused to give a polypeptide unit of between 10 and 1000 residues, preferably between 25 and 100 residues. By fusion of this nucleic acid fragment with that encoding a phage protein, for example pIII of the bacteriophage fd, the fusion molecule may be displayed on the surface of phage. Screening of the phage library with serotonin will then identify those clones of interest. These clones can then be subjected to iterative rounds of mutagenesis and screening to improve the affinity of the generated molecules for histamine or serotonin.

Residues with analogous physical- properties to those that comprise the serotonin binding site may also form part of a molecule according to the present invention. For example, with respect to the protein FS-HBP2, either of the charged residues glutamate or aspartate may occupy position 39 and 82 in the sequence. At position 108 in the sequence, it is envisaged that any hydrophobic amino acid residue may occupy this site, provided that steric concerns are satisfied with respect to the molecular configuration of the binding site. Phenylalanine, isoleucine and leucine are preferable residues at this position. At position 42, tryptophan is preferred.

Additionally, at position 100 in the histamine binding compound sequence, it is preferred that a tyrosine residue is present. This molecule is thought to contribute to the stability of histamine in the binding site. Any molecular structure that retains this amino acid side-chain or an equivalent in this position forms an aspect of the present invention.

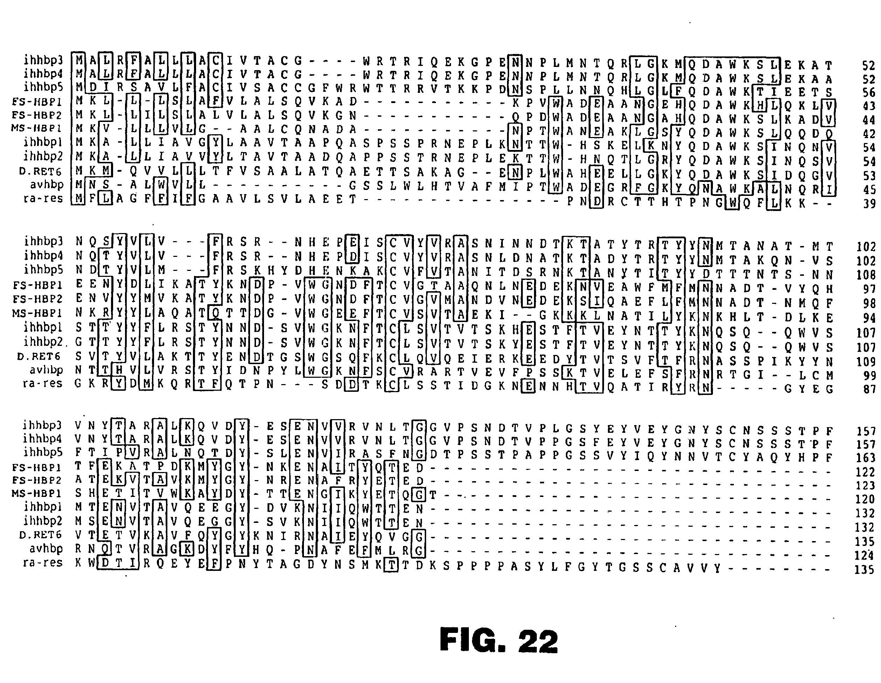

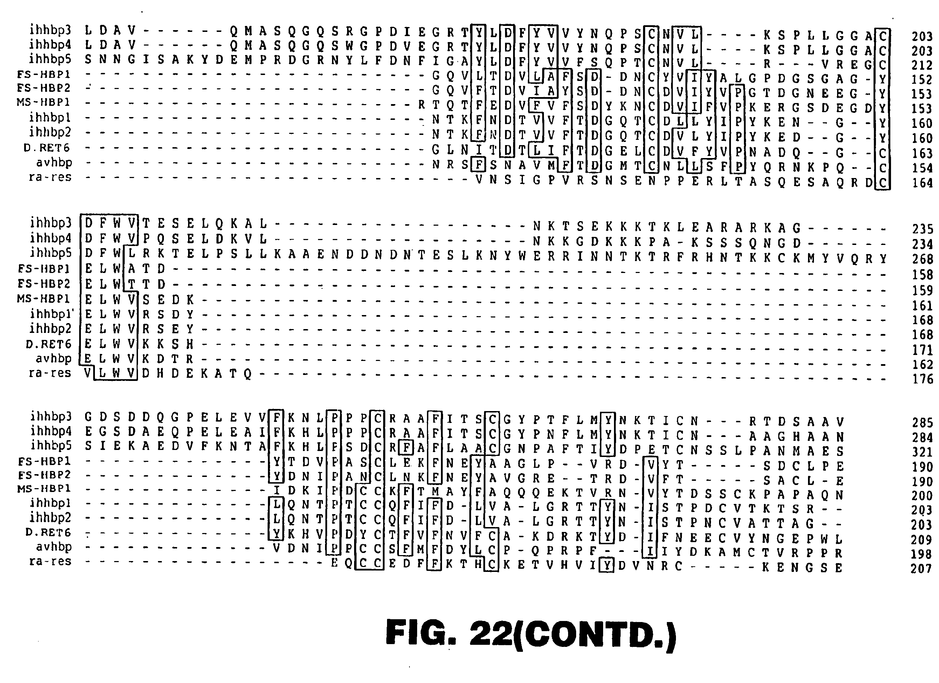

Due to variations in the length and sequence of the four proteins explicitly described herein, the method of numbering residues differs between proteins. However, it will be apparent from the alignment shown in Figure 22 which residues correspond to the residues numbered according to the sequence of FS-HBP2.

It is envisaged that proteins according to the present invention may be stabilised by the presence of disulphide bridges in the structure. For example, the cysteines found in positions 48, 169, 119 and 148 of FS-HBP2 are conserved in all four histamine binding proteins identified so far. Two disulphide bridges are formed in FS-HBP2, one between cysteines 48 and 169, the other between 148 and 119. Accordingly, for any protein fragment designed to mimic the structure of the natural histamine or serotonin binding compound binding site, these cysteine residues may be present in the sequence so that one or both disulphide bridges form within the protein structure.

It is preferred that in addition to the high affinity with which the compounds of the present invention bind to serotonin, this binding phenomenon is also specific for serotonin. The advantages that this specificity confer on the compounds will be obvious to the man of skill in the art.

For many applications, compounds according to the present invention may be fused to an effector or reporter molecule such as a label, toxin or bioactive molecule. Such molecules may comprise an additional protein or polypeptide fused to the histamine or serotonin binding compound at its amino- or carboxy-terminus or added internally. The purpose of the additional polypeptide may be to aid detection, expression, separation or purification of the histamine or serotonin binding compound or may be to lend additional properties to the compound as desired.

Particularly suitable candidates for fusion will be reporter molecules such as luciferase, green fluorescent protein, or horse radish peroxidase. Labels of choice may be radiolabels or molecules that are detectable spectroscopically, for example fluorescent or phosphorescent chemical groups. Linker molecules such as streptavidin or biotin may also be used. Additionally, other peptides or polypeptides may be fused to a histamine or serotonin binding compound. Suitable peptides may be, for example, β-galactosidase, glutathione-S-transferase, luciferase, polyhistidine tags, secretion signal peptides, the Fc region of an antibody, the FLAG peptide, cellulose binding domains, calmodulin and the maltose binding protein. Antibodies or peptides used to target the histamine or serotonin binding compounds more efficiently towards a site of action (for example antibodies against membrane proteins of mast cells) may also be fused to the histamine or serotonin binding compounds.

These fusion molecules may be fused chemically, using methods such as chemical cross-linking. Suitable methods will be well known to those of skill in the art and may comprise for example, cross-linking of the thiol groups of cysteine residues or cross-linking using formaldehydes. Chemical cross-linking will in most instances be used to fuse non-protein compounds, such as cyclic peptides and labels.

When it is desired to fuse two protein molecules, the method of choice will often be to fuse the molecules genetically. In order to generate a recombinant fusion protein, the genes or gene portions that encode the proteins or protein fragments of interest are engineered so as to form one contiguous gene arranged so that the codons of the two gene sequences are transcribed in frame.

The compounds of the present invention may also comprise serotonin binding compounds bound to a support that can be used to remove, isolate or extract serotonin from body tissues, blood or food products. The support may comprise any suitably inert material and includes gels, magnetic and other beads, microspheres, binding columns and resins.

If proteinaceous, the serotonin binding compound may be derived from any organism possessing a protein in the same family as the serotonin binding compounds identified to date. By protein family is meant a group of polypeptides that sham a common function and exhibit common sequence homology between motifs present in the polypeptide sequences. By sequence homology is meant that the polypeptide sequences are related by divergence from a common ancestor.

Preferably, proteins or protein fragments are derived from blood-feeding ectoparasites, spiders, scorpions or snakes or other venomous animals. More preferably, the proteins or protein fragments are derived from ticks, most preferably Ixodid ticks such as, for example, *Rhipicephalus appendiculatus.*

Most preferably, proteinaceous compounds according to the present invention are derived from any one of the proteins FS-HBP1, FS-HBP2, MS-HBP1, D.RET6.

Protein or peptide compounds according to the invention will preferably be expressed in recombinant form by expression of the encoding DNA in an expression vector in a host cell. Such expression methods are well known to those of skill in the art and many are described in detail in *DNA cloning: a practical approach, Volume II: Expression systems,* edited by D.M. Glover (IRL Press, 1995) or in *DNA cloning: a practical approach, Volume IV: Mammalian systems,* edited by D.M. Glover (IRL Press, 1995). Protein compounds may also be prepared using the known techniques of genetic engineering such as site-directed or random mutagenesis as described, for example, in *Molecular Cloning: a Laboratory Manual*: 2nd edition, (Sambrook *et al.,* 1989, Cold Spring Harbor Laboratory Press) or in *Protein Engineering: A practical approach* (edited by A.R Rees *et al.,* IRL Press 1993).

Suitable expression vectors can be chosen for the host of choice. The vector may contain a recombinant DNA molecule encoding compounds of the present invention operatively linked to an expression control sequence, or a recombinant DNA cloning vehicle or vector containing such a recombinant DNA molecule under the control of a promoter recognised by the host transcription machinery.

Suitable hosts include commonly used prokaryotic species, such as *E. coli,* or eukaryotic yeasts that can be made to express high levels of recombinant proteins and that can easily be grown in large quantities. Mammalian cell lines grown *in vitro* are also suitable, particularly when using virus-driven expression systems such as the baculovirus expression system which involves the use of insect cells as hosts. Compounds may also be expressed *in vivo,* for example in insect larvae or in mammalian tissues.

Various aspects and embodiments of the present invention will now be described in more detail by way of example, with particular reference to proteinaceous histamine or serotonin binding compounds isolated from ticks. It will be appreciated that modification of detail may be made without departing from the scope of the invention.

### Brief description of Figures

Figure 1 is the sequence of FS-HBP1 (SEQ. ID. 1), showing sequencing primers and sequencing strategy.
Figure 2 is the sequence of FS-HBP2 (SEQ ID 2), showing sequencing primers and sequencing strategy.
Figure 3 is the sequence of MS-HBP1 (SEQ ID 3), showing sequencing primers and sequencing strategy.
Figure 4 is the sequence of D.RET6 (SEQ ID 4), showing sequencing primers and sequencing strategy.

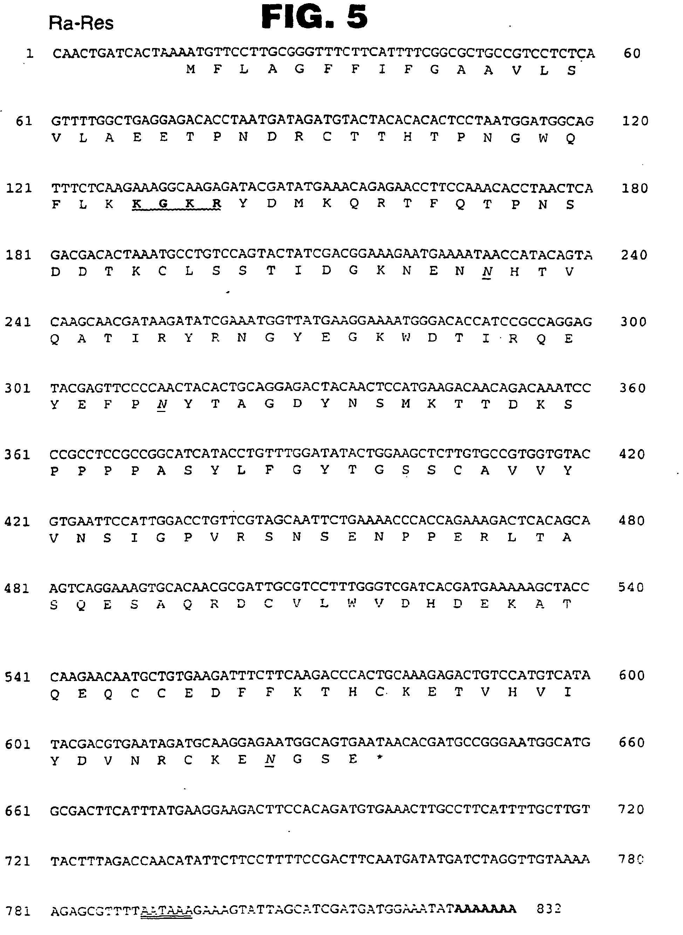

Figure 5 is the sequence of ra-RES (SEQ. ID. 5). Asparagines that are part of putative glycosylation recognition sites are underlined and shown in italics. The squiggly line denotes a possible amidation site I; the double line indicates the putative polyadenylation signal and the polyA-tail is shown in bold letter type.
Figure 6 is the sequence of Av-HBP (SEQ. ID. 6). Sequence of the Av-HBP cDNA and its inferred primary structure. The cDNA has a remarkably long non-coding region, downstream of the stop codon.
Figure 7 is the sequence of Ih/Bm-HBP1 (SEQ. ID. 7).
Figure 8 is the sequence of Ih/Bm-HBP2 (SEQ. ID. 8).
Figure 9 is the sequence of Ih/Bm-HBP3 (SEQ. ID. 9).

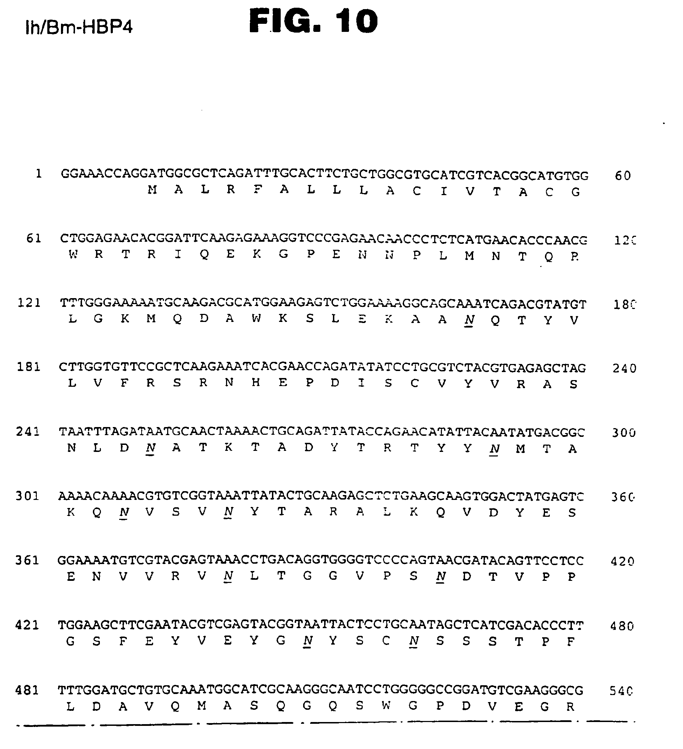

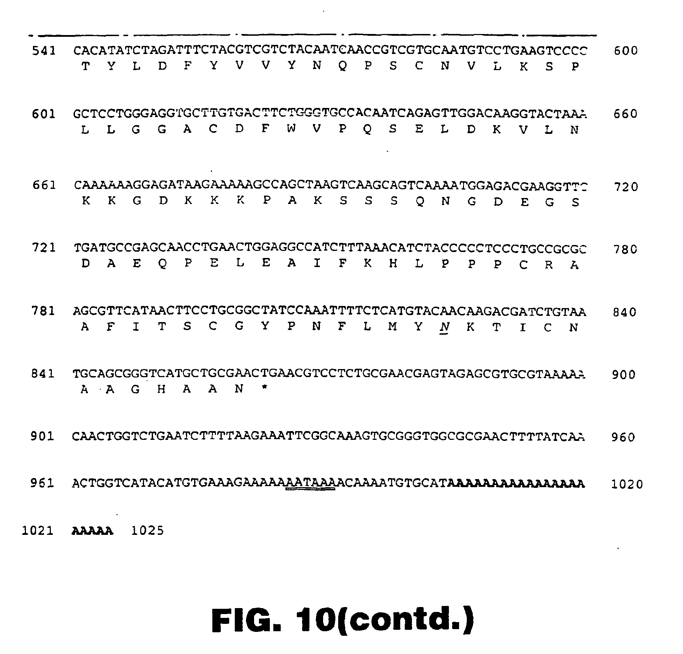

Figure 10 is the sequence of Ih/Bm-HBP4 (SEQ. ID. 10).

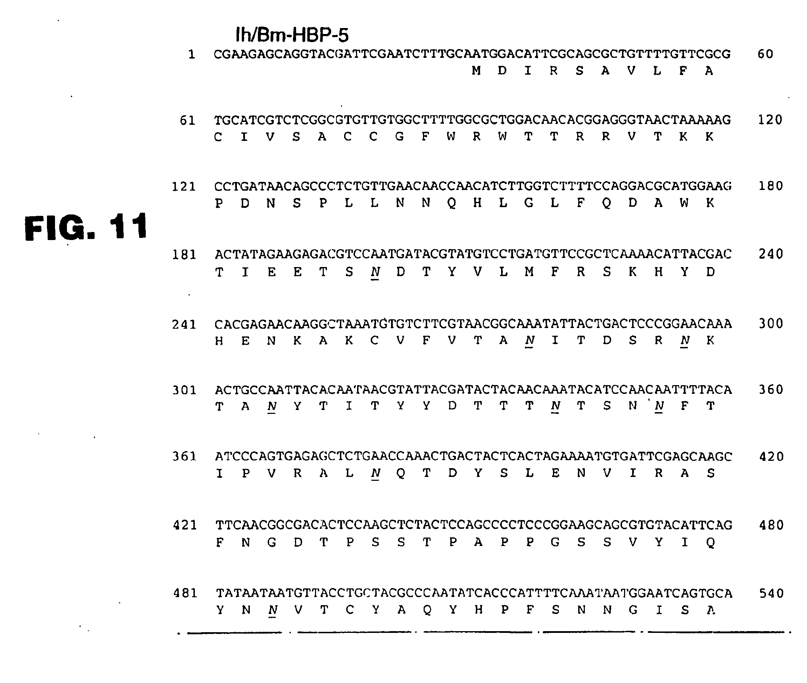

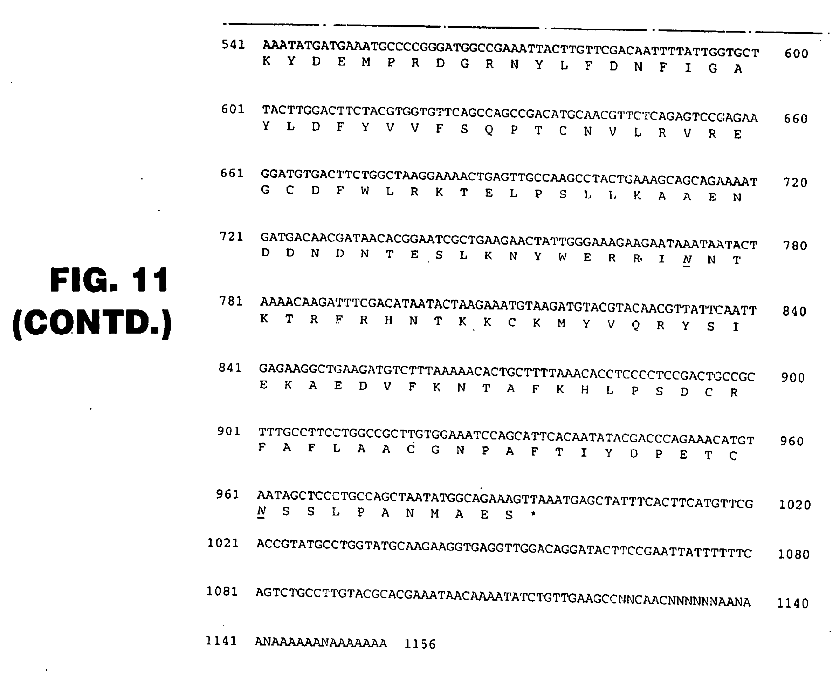

Figure 11 is the sequence of Ih/Bm-HBP5 (SEQ. ID. 11).

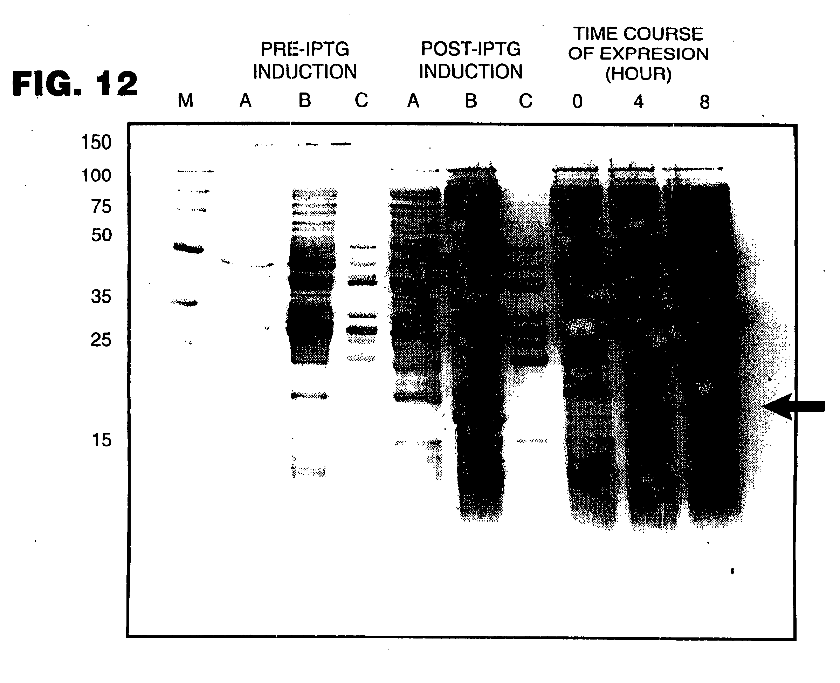

Figure 12 is a silver-stained 12% SDS-polyacrylamide gel showing fractions obtained from a nickel resin column used to purify recombinant D.RET6 expressed in insect cells. A, flow-through fraction; B, first wash fraction (10 volumes of phosphate buffer pH 6.5); C, second wash fraction (with 5mM imidazole); D, third wash fraction (with 10mM imidazole); E, fourth wash fraction (with 15 mM imidazole); F, fifth wash fraction (with 20 mM imidazole); G, elution fraction with 300mM Imidazole: H, purified D.RET6 after ion exchange chromatography.
Figure 13 is a silver-stained 12% SDS-polyacrylamide gel of D.RET6 overexpressed in insect cells. Lanes A and B shows purified D.RET6 as a monomer (25 kDa), dimer (50 kDa) and trimer (85 kDa) and lane C is after deglycosylation. Cleaved oligosaccharides were retained in the stacking gel and can be seen at the top of lane C. Excess PNGase-F appears as a band of approximately 30 kDa. Lane D shows the purified monomeric form of D.RET6.
Figure 14 shows Western blot detection of native histamine-binding protein in *Dermacentor reticulatus* salivary gland extract. Lane A, non-reduced form; lane B, reduced form; lane C, deglycosylated form.
Figure 15: Determination of the IC₅₀ of three unlabeled competitors e.g. histamine, 1-methylhistamine, and 3-methylhistamine by generating a competitive binding curve of recombinant D.RET6. The graphically derived of IC₅₀ of the unlabelled ligands above in displacing the radioactive histamine from the binding site by 50% are 133 nM, 750 µM and 365µM, respectively.
Figure 16 shows saturation curves of histamine binding in the absence (solid line) or presence (dashed lines) of serotonin. ◆no serotonin, ■2.4 µM and Δ 23.8 µM serotonin, respectively.

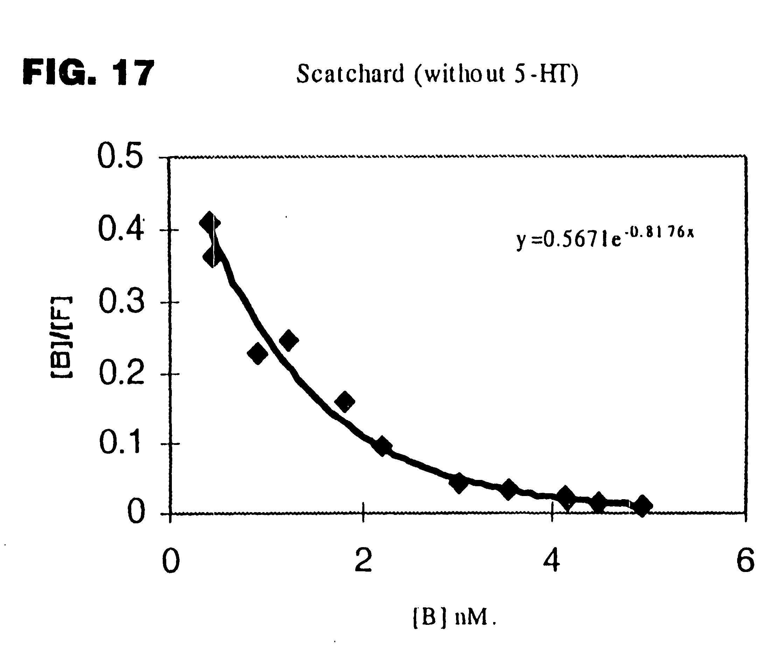

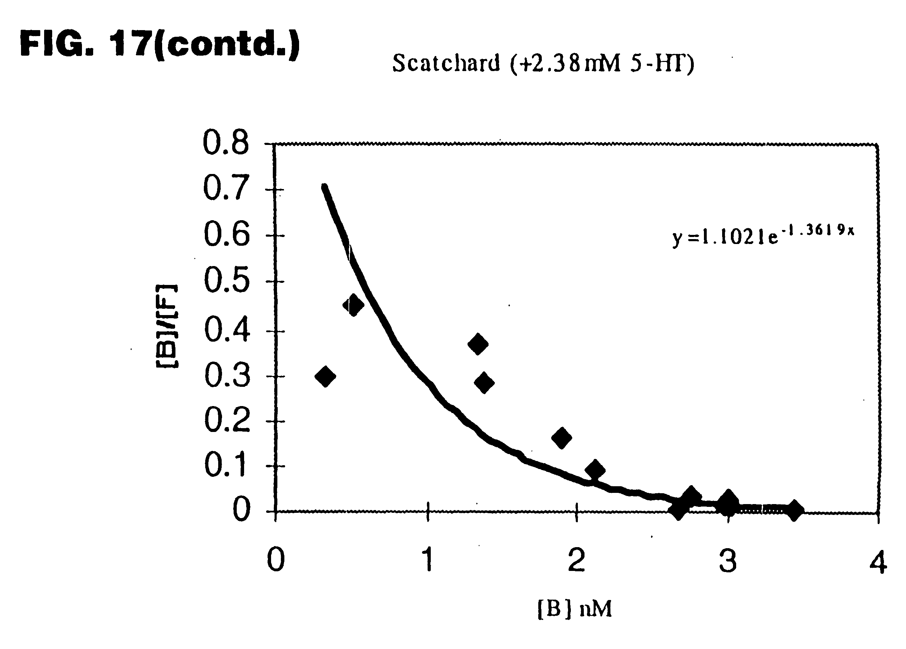

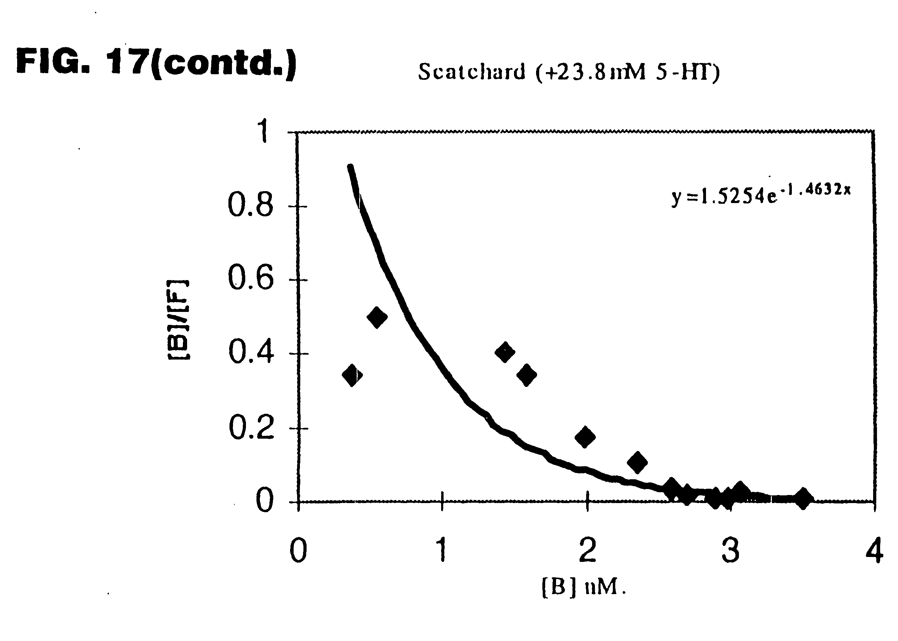

Figure 17: Radioactive histamine-binding assay of the recombinant D.RET6 in the absence (A) and presence of serotonin at 2.4 µM (B) and 23.8 µM (C).

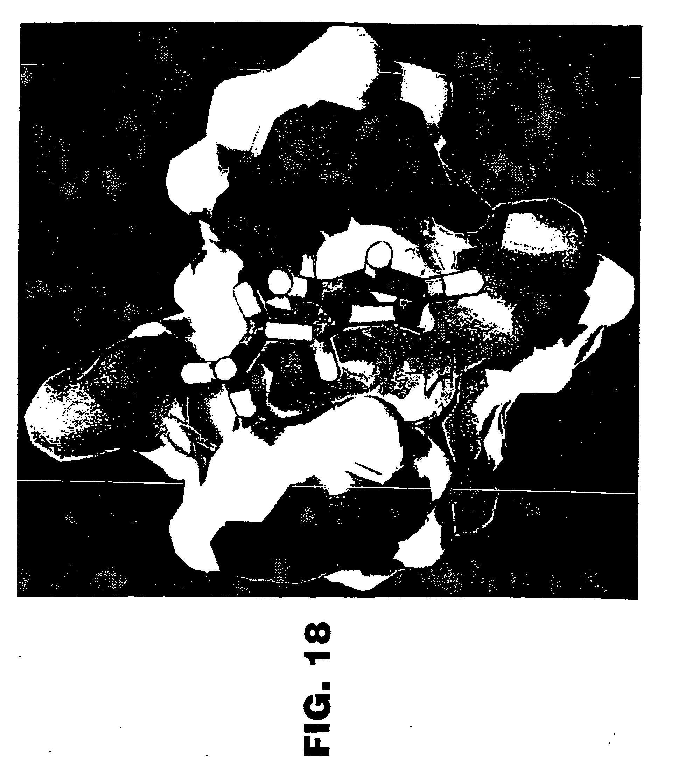

Figure 18: Depiction of Histamine bound in the binding pocket of the histamine binding site of a cyclic peptide.

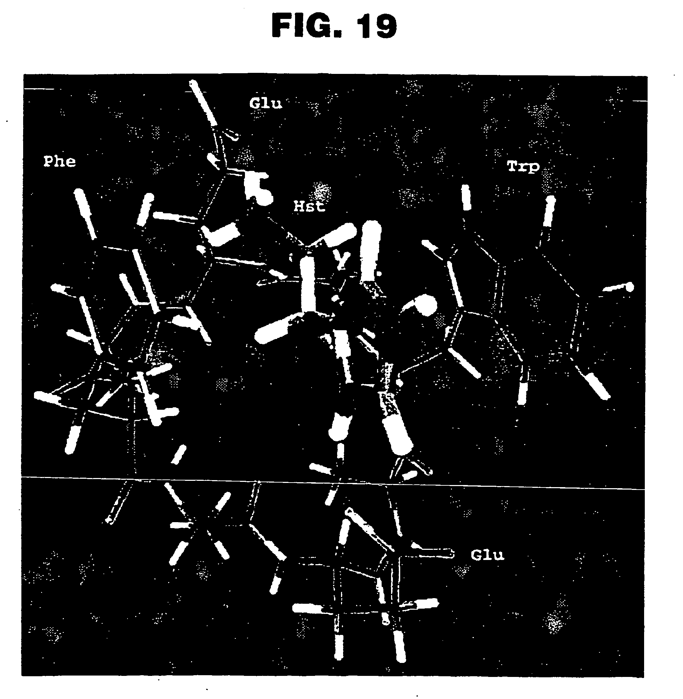

Figure 19: Three-dimensional depiction of Histamine bound in the binding pocket of the histamine binding site of a cyclic peptide.

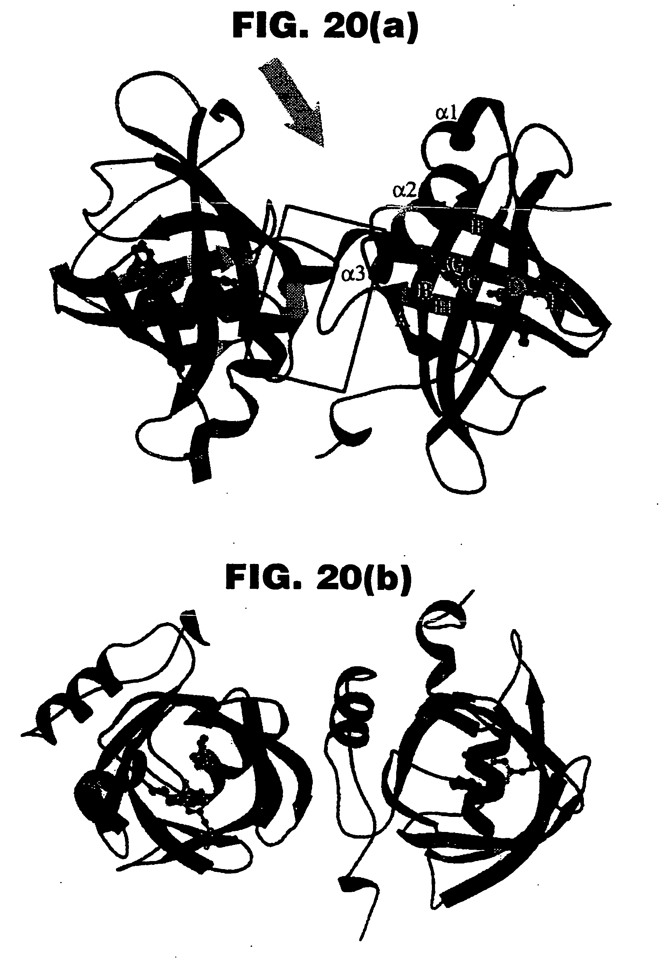

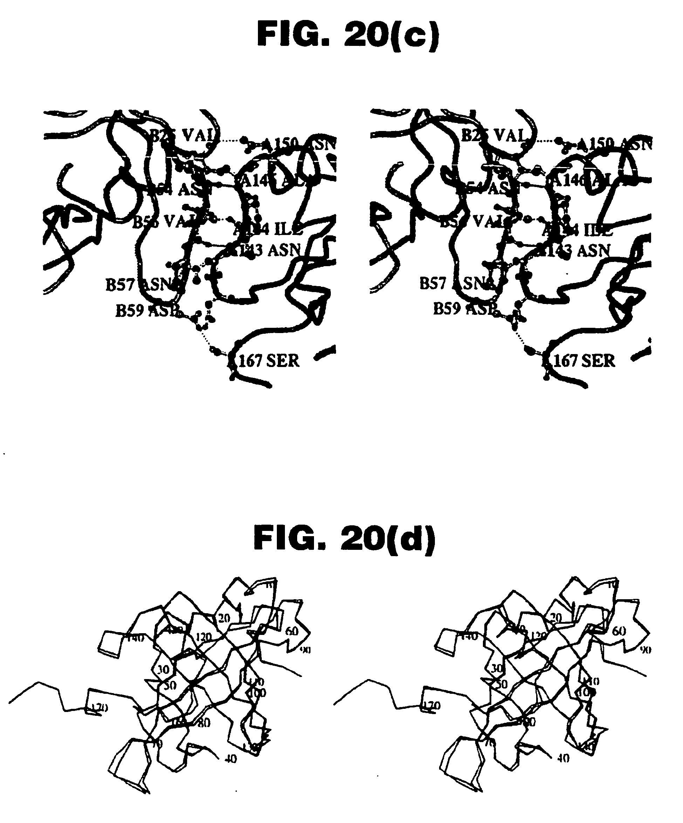

Figure 20: (a) Ribbon diagram showing the arrangement of molecules A and B in the asymmetric unit of FS-HBP2. The 13 sheet is labelled A - I and α helices 1-3. The histamine ligands are shown as ball-and-stick representation, along with Tyr100. The arrow indicates the direction of view in (b) and the boxed region shows the portion enlarged in (c). (b) This view shows the helix and extended loop which occlude the barrel entrance. (c) Stereo view of the individual hydrogen bond contacts between residues in the A and B molecules. (d) Superposed C-α traces of molecules A and B [performed using SHP, (Stuart *et al.* 1979)].
Figure 21: Stereo views of the *H* (a), *L* (b) and *apo-L* (c) histamine binding sites, respectively. In each case the 2|Fo|-|Fc| map is displayed around the ligand. The figure shows the core structure of the protein (tube); contacting residues and secondary structure elements are labelled. In (c) the bound structure L (drawn transparent), has been superposed onto the apo coordinates.
Figure 22: An alignment of the cDNA-inferred amino acid sequences of the various HBPs, created using the pileup commands of the Genetics Computer Group. (1994). Program Manual for the Wisconsin Package, version 8. (575 Science Drive, Madison, Wisconsin, USA 53711).

### EXAMPLES

### Ticks

Ticks were reared according to Jones *et al.* (1988), as described in detail in co-pending International patent application PCT/GB97/01372, the contents of which are incorporated herein in their entirety.

The identification of proteins FS-HBP1, FS-HBP2, MS-HBP1 and D.RET6, and cloning of the encoding genes is also described in PCT/GB97/01372 (see Examples 1, 2, and 3).

The Ra-d0 (*Rhipicephalus appendiculatus*) library was constructed with mRNA from salivary glands of unfed *R. appendiculatus* ticks (127 males, 124. females). The Av-library (*Amblyomma variegatum*) was constructed with mRNA isolated from salivary glands of partially fed, adult *A. variegatum* ticks (50 males, fed for 6-7 days, and 50 females, fed for 4 to 5 days). The Ih/Bm library (a mixed *Ixodes hexagonus* and *Boophilus microplus* library) was constructed with the pooled mRNA from salivary glands of partially fed *Boophilus microplus* (30 females) and three-day fed *Ixodes hexagonus* ticks (20 males/20 females).

### Example 1: Cloning and sequencing of the Amblyomma variegatum, Ixodes hexagonus and Boophilus microplus libraries

Using the RNAce Total Pure extraction kit (Bioline Ltd., UK), total RNA was isolated from salivary glands of partially fed *Amblyomma variegatum, Ixodes hexagonus* and *Boophilus microplus* ticks. The RNA samples were submitted to reverse-transcriptase polymerase chain reactions (RT-PCR), using the Titan One-Tube RT-PCR system (Boehringer Mannheim) and-degenerate primers:
5'-AAYGGNGARCAYCARGAYGCNTGGAA; and
5'-KTRTMRTCNGTNRYCCANARYTCRTA, the design of which was based on conserved domains in the *Rhipicephalus appendiculatus* HBPs.

Cycling conditions were according to the manufacturer's suggestions, but a 48 °C annealing temperature was used. RT-PCR products were ligated into the pGEM-T vector (Promega), and partially sequenced, using the SP6 and T7 primer sites flanking the cloning site. Inserts of which the inferred amino-acid sequences showed similarity to the original *Rhipicephalus* HBPs were labeled with digoxygenin, using the High Prime DNA labeling kit (Boehringer Mannheim), and were used as probes to screen the libraries. An anti-digoxigenin antibody conjugated with alkaline-phosphatase was used to visualize probe hybridization. The Ra-d0 library was screened (according to Mierendorf et al., 1987) with serum from a guinea pig that had acquired resistance against *R. appendiculatus* ticks following repeated infestations.

The pBluescript SK (-) phagemids of positive clones were excised *in vivo,* using the R408 helper phage, as described by Short et al. (1988).

### Example 2: Recombinant protein expression

Expression and purification of FS-HBP1, FS-HBP2, MS-HBP1 is described in detail in Example 3 of PCT/B97/01372.

### 1) Construction of clones for D.RET6

Using an *E. coli*-based expression system, the DNA sequence encoding D.RET6 (from Glu29 to Leu109) was subcloned as a *Bcl*I/ *Xho*I fragment into *BamH*I /*Xho*I -digested pET-23 a (+) in the same reading frame as the 6x His tag using the PCR technique with the following primers, 5'-TATATGATCAGAAAACCCGCTCTGGG-3' and 5'TATA CTCGAGCCA GGGTTCGCCGT-3' (the enzyme recognition sites are underlined.). The recombinant plasmid was transformed into host strain AD494(DE3) pLysS, which uses the T7 system, and success of the procedure was confirmed by sequencing.

D.RET6 was also expressed in bacteria. The bacterial transformant was grown at 37°C in Luria-Bertani medium containing ampicillin and chloramphenicol. The culture was induced at its exponential growth phase (OD₆₀₀ about 0.5) using 0.5-1 mM isopropyl β-D-thiogalactopyranoside (IPTG) and grown further for at least 4 hours before harvesting by centrifugation (4500xg, 4 °C, 5 mins.). The pellet was resuspended in the lysis buffer containing 6M urea, 20mM Tris pH8 and 500mM NaCl, sonicated briefly on ice, a few drops of Triton-X 100 added, and mixed by rolling at 4 °C for 5 mins. The supernatant was collected by centrifugation at 7500xg 4 °C for 30 mins.

D.RET6 was also expressed in the baculovirus expression system. The DNA fragment containing the complete coding sequence of D.RET6 was amplified using the oligonucleotides: 5'-TATGAAGATGCAGGTAGTGC-3' and:
5'-ATATGATCAGCCAGGGTTCGCCGT-3'.

The resulting PCR product was blunt-ended, digested with *Bcl*I, and ligated with the transfer vector pAcCL 29-1 -6xHis (Livingstone, 1989), which was prepared to have a blunt end at a *Sac* I site and an adhesive end at a *BamH*I site. The ligation product was then transferred into *E. coli* (XL 1-Blue) and the transformant was grown to produce the plasmid. The plasmid was checked for the absence of any undesired mutations by complete resequencing. Sf9 cells were cotransfected at different ratios with the recombinant transfer vector and *Bsu36*I-cut *Autographa californica* polyhedrosis virus (BacPAK6) DNA in the presence of 8 µg of Lipofectin (Gibco BRL) per 24 µl reaction.

For expression, recombinant baculoviruses were identified as galactosidase-negative plaques by plating under Seaplaque agar. The putative recombinant clones were plaque purified once more. The clone from an infected Sf21 cell lysate that gave a positive result on a western blot using polyclonal anti-bacterial expressed D.RET6 antisera was amplified and used in the production of the recombinant protein. For each subsequent production of the fusion protein, Sf9 cells were infected with these baculoviruses at MOI of about 5 and grown for 2-3 days before collecting the supernatant by centrifugation at 1000xg for 5 min. After 60% ammonium sulphate precipitation of the supernatant, the pellet was discarded. The pellet obtained at 100% ammonium sulphate precipitation was redissolved in Buffer A (50mM sodium phosphate, 300mM NaCl and 10% glycerol, pH8).

### 2) Protein purification and production of antisera

The purification steps were the same for both bacterial and baculovirus expression systems. Briefly, Ni²⁺-nitrilo-triacetic acid resin, previously equilibrated in buffer A, was added to the supernatant or the solution. After incubation on a roller (2 hr 4 °C ), the resin was transferred to a column and washed with 20 volumes of phosphate buffer (50mM sodium phosphate, 300mM NaCl and 10% glycerol, pH6.5). Protein was eluted with 5 volumes of 300mM Imidazole pH 8, concentrated, and the solvent replaced with phosphate-buffered saline using a Centricon-10 (Amicon) filtration unit. The recombinant D.RET6 was further purified on a HiTrap SP column (Pharmacia) using a gradient of 0.1-0.6 M NaCl in 50 mM MES pH 6.2.

To determine the location and solubility of the expressed protein in *E. coli,* the culture of the bacterial transformant was sampled before and 4 hours after IPTG-induction and processed according to the protocol described in the Qiaexpressionist booklet (QIAGEN). In brief, the sample was spun down, lysed by a cycle of freezing and thawing, and brief sonication, then centrifuged to separate insoluble proteins and the cytosolic soluble protein fraction. In another sampled culture, cells was pelleted and subjected to osmotic shock by, first, resuspending in 30mM Tris solution containing 20% sucrose (pH8) and incubating at room temperature with shaking in the presence of 1mM EDTA. The cells were then collected and resuspended in the low-osmotic ice-cold solution (5mM MgSO₄). The proteins released in the solution were collected by centrifugation. All fractions together with the pre-induced fraction were analysed by 12% SDS-PAGE.

Preparation of fusion proteins for antibody production and the technique for immunizing with small amounts of antigen are described by Sambrook *et al,* 1989. Briefly, guinea-pig anti-D.RET6 antiserum was prepared by repeated (x3) intraperitoneal immunization of guinea pigs with homogenized 10% SDS-PAGE gel slices containing microgram quantities of bacterially-expressed purified D.RET6 in phosphate-buffered saline.

### 3) Electrophoresis and Western Blotting

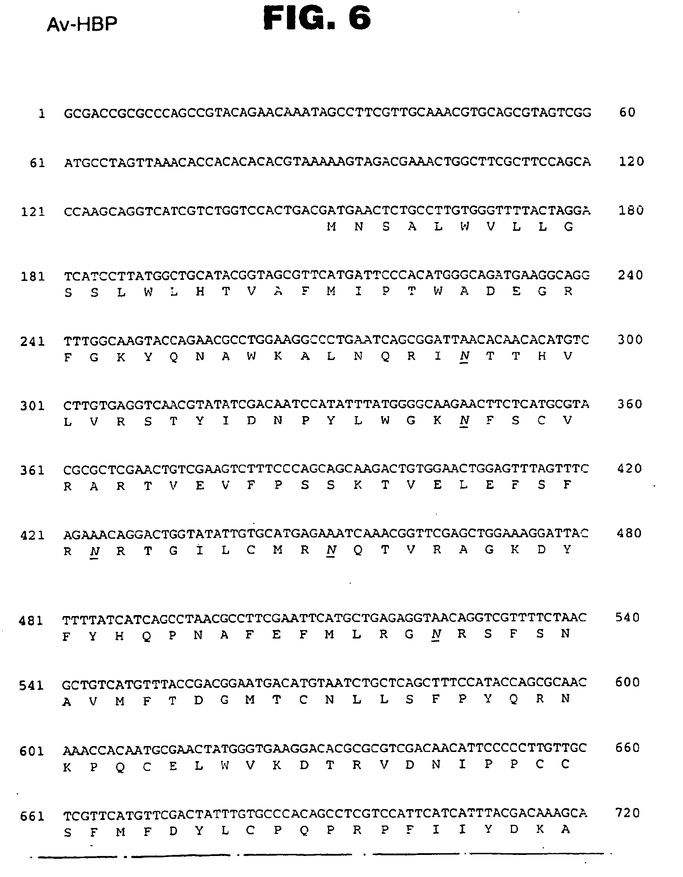

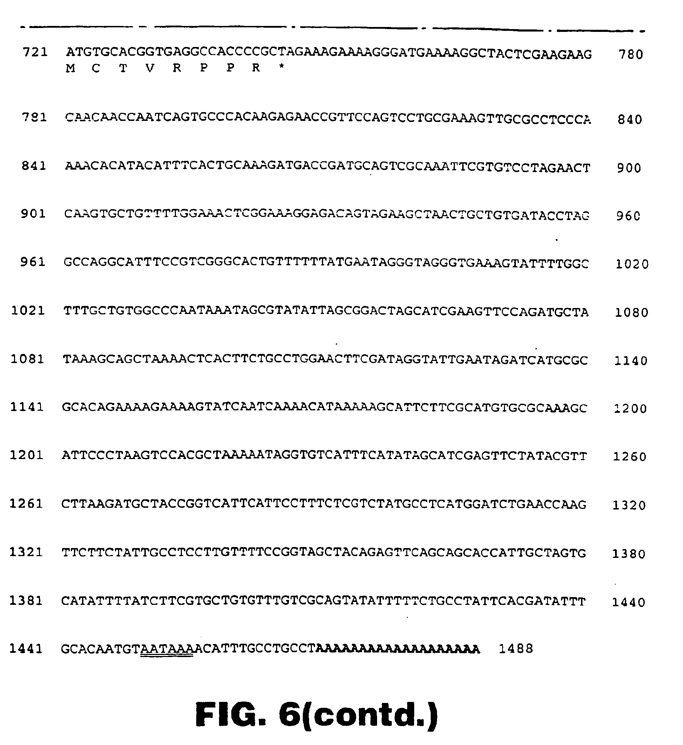

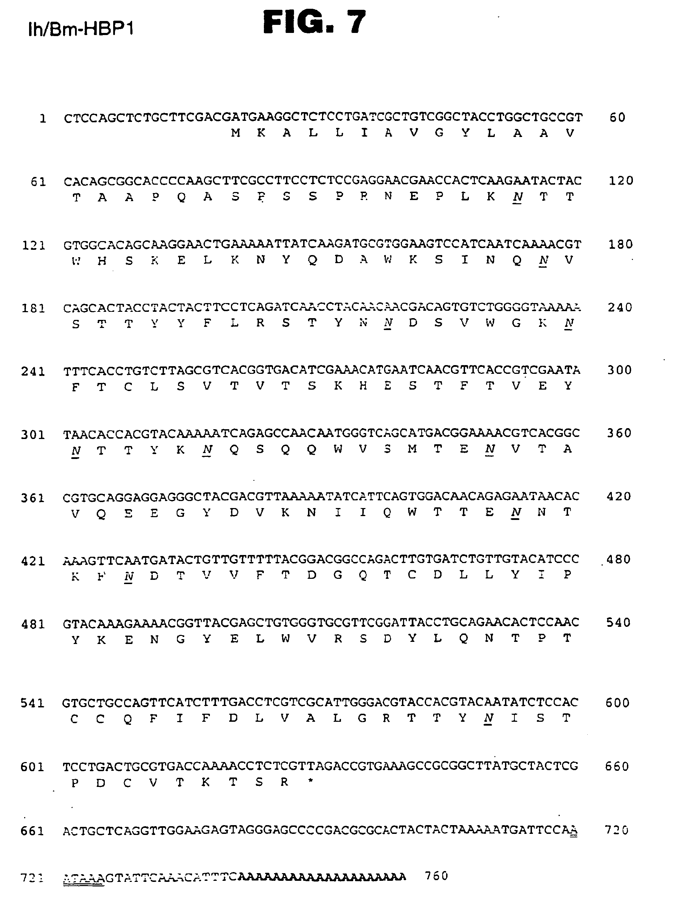

SDS-PAGE gels and Western blots showing expression of FS-HBP1, FS-HBP2, MS-HBP1 is demonstrated in PCT/GB97/01372(see Figures 6 and 7).

Salivary glands (and other tissues) were excised from ticks at different time points of the feeding period, and homogenised in PBS. The homogenates were centrifuged at 10,000g for 5 minutes and the supernatants were submitted to sodium dodecyl sulphate-polyacrylamide electrophoresis (SDS-PAGE; Laemmli, 1970).

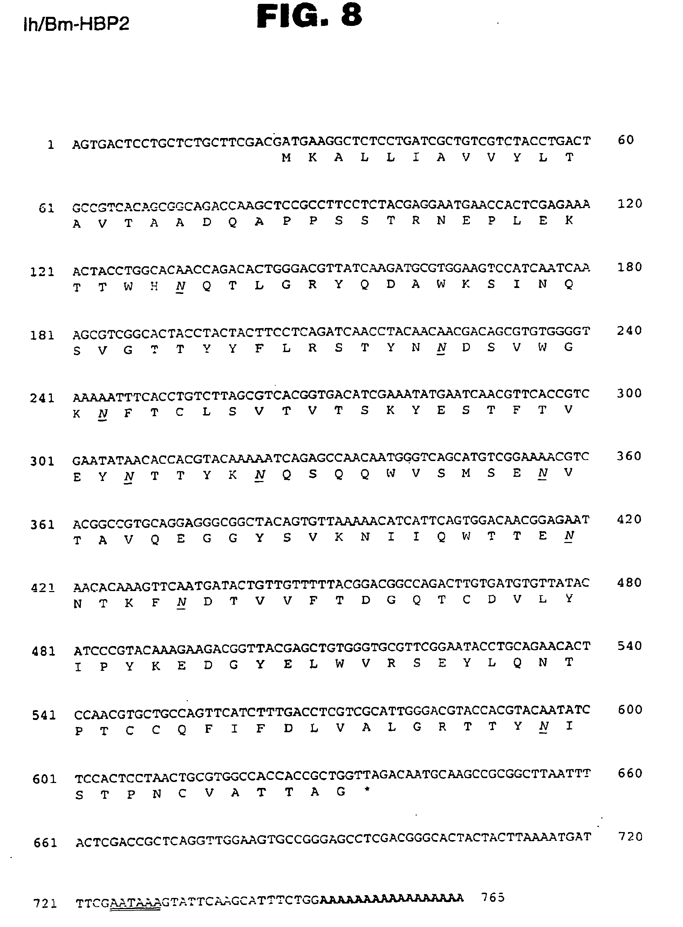

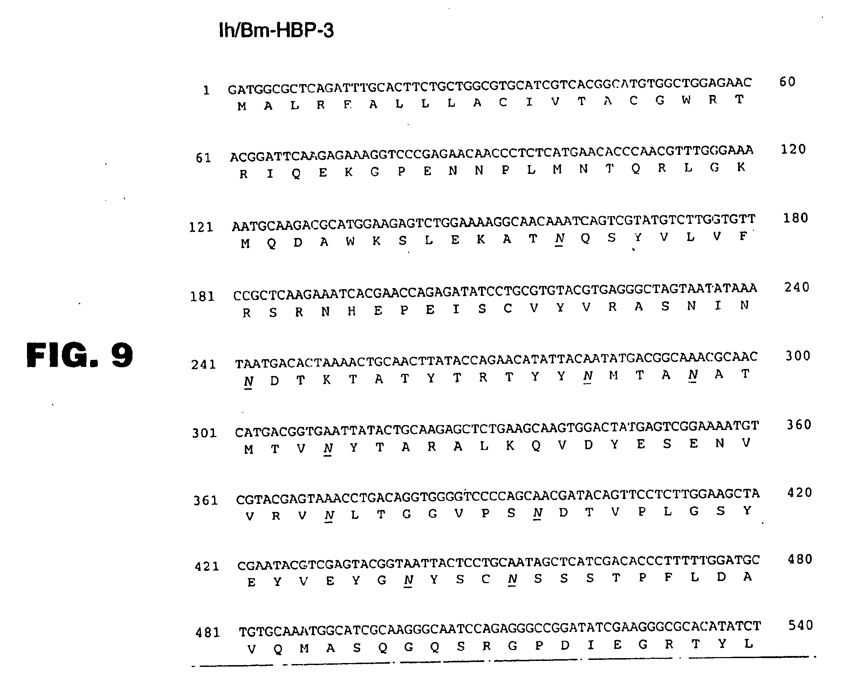

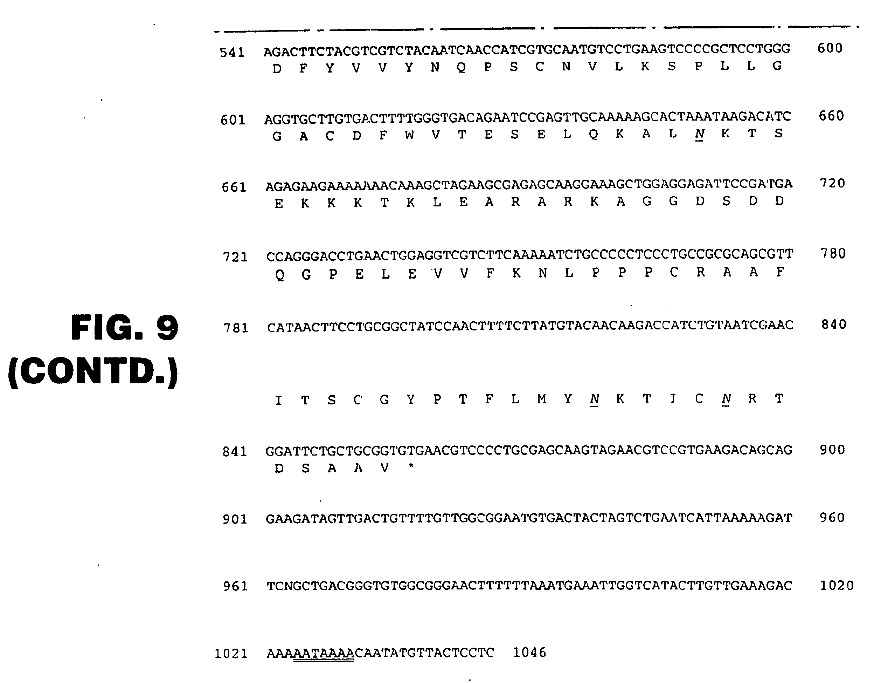

Figures 8 and 9 show the expression of D.RET6 in *E. coli* and insect cells, respectively.

For Western blotting, proteins were transferred to nitrocellulose (Gelman Sciences) by means of semi-dry electroblotting (Kyhse-Anderson, 1984) using an AE-6675 Horizblot MS-HBP1 from the first day p.a. until the end of the feeding period.

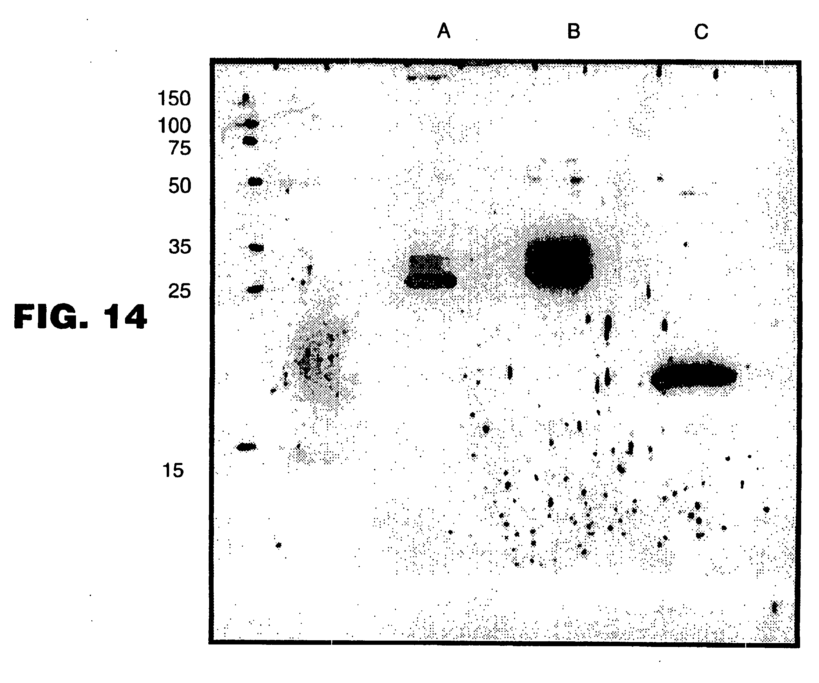

Figure 14 shows a Western blot showing expression of the D.RET6 protein.

### 4) Av-HBP protein expression and purification

The coding region of the Av-HBP cDNA was PCR amplified (95 °C for 30", 50 °C for 30", 72 °C for 30"; 18 cycles) using a forward primer designed to add a *Sac* I site upstream of the start codon (5'- TATGAGCTCATGAACTCTGCCTTGTGG; the *Sac*I site is underlined), and a reverse primer (5'- TATGGATCCGGGGTGGCCTCACCG) containing a *Bam*HI site (underlined). The product was ligated in between the *Sac* I and *Bam*HI sites of the pAcC129.1 transfer vector (Livingstone and Jones, 1989) which had been modified by the insertion of six histidine codons and a stop codon, downstream of the *Bam*HI recognition site (see original patent). This resulted in the addition of the sequence Ile-(His)₆ to the carboxy- terminus of Av-HBP.

Co-transfection of Sf21 Spodoptera cells with the transfer vectors and baculovirus (BacPak6), and amplification of recombinant virus, were according to Kitts and Possee (1993). *Trichoplusia ni* cells (High Five, Invitrogen) and TC100 medium containing 10% foetal bovine serum (Gibco BRL) were used for protein expression. After 60 hours incubation at 28 °C, the secreted protein was precipitated from the medium with ammonium sulphate (in the 45-80 % saturation fraction) and redissolved in a 50 mM sodium phosphate buffer (pH 8.0), containing 300 mM NaCl and 10% glycerol (buffer A). Talon beads (Clontech) were added to capture the oligohistidine-tagged proteins (1 hour incubation at 4°C). The beads were applied to a 10 ml-column, and washed with buffer A (20 volumes), then with 50 mM sodium phosphate buffer (pH 7.0), containing 300 mM NaCl and 10% glycerol (20 volumes), and finally with 50 mM sodium phosphate buffer (pH 7.0; 20 volumes). The protein was eluted using 200 mM imidazole in sodium phosphate buffer (100 mM, pH adjusted to 8.0).

### Example 3: Characterisation of proteins

### 1) Histamine binding assays

The purified recombinant proteins were submitted to histamine binding assays as set out in Warlow and Bernard (1987). This method used protein precipitation to separate free from bound ligand (radiolabelled histamine) by addition of polyethylene glycol (molecular weight 8000) and centrifugation. In all experiments, thin-layer chromatographs were run in an acetate-ammonia solvent system after a four hour incubation period to ensure that no metabolisation of histamine had occurred.

Saturable binding of ³H-histamine was obtained with FS-HBP1, FS-HBP2, MS-HBP1 rHBPs (see Figure 20 of PCT/GB97/01372).

For Av-HBP, semi-purified recombinant protein was incubated with varying amounts of tritiated histamine, and the amount of bound radiolabel was determined by liquid scintillation counting. Separation of free from bound ligand was obtained following the method described by Warlow and Bernard (1987), which uses polyethylene glycol (PEG 8000) to precipitate the protein. Results (not shown) suggested that the equilibrium dissociation constant (Kd) for histamine is around 7.3nM.

### 2) Radioligand binding assay for D.RET6

The recombinant D.RET6 was diluted with 1.5% γ-globulin (Sigma) and used in one set of experiments. Fifty microlitres of the protein solution were incubated with 50 µl of 1:2500 dilution of [2,5-³H] histamine diHCl (1µCi/µl) solution (Amersham) at room temperature for at least 3 hours with or without increasing concentrations of unlabelled histamine. All the assays were carried out in a total volume of 200 µl. The incubations were terminated by adding 125 µl of PEG 8000 (36% w/v in PBS) and centrifuged in a microfuge at maximum speed for 12 minutes to collect the bound protein. The tubes were spun once more to remove all supernatant without disturbing the pellets. Subsequently, pellets were redissolved in PBS. Three millilitres of liquid scintillation cocktail (Beckman) were added and the radioactivity measured using a liquid scintillation counter (Wallac, 1217 Rackbeta).

In a second set of experiments, a competitive binding assay was used to compare three unlabelled competitor ligands (histamine, 1-methylhistamine and 3-methylhistamine).

To study the effect of serotonin on histamine binding activity, 10 µl of PBS (as a control experiment) or 10 µl serotonin (50 µM or 500 µM) were added to each 200 µl binding assay.

For data analysis, the ligand affinity constant was estimated from Scatchard plots as previously described by Hulme, 1992, *Receptor-Ligand Interactions, IRL Press, Oxford).* The nonlinear regression was used to fit the data (Motulsky, 1987, *FASEB J,* **1***:* 365-374) and two asymtotic straight lines were made as described elsewhere (Feldman, 1972, *Analytical Biochem* **48**: 317).

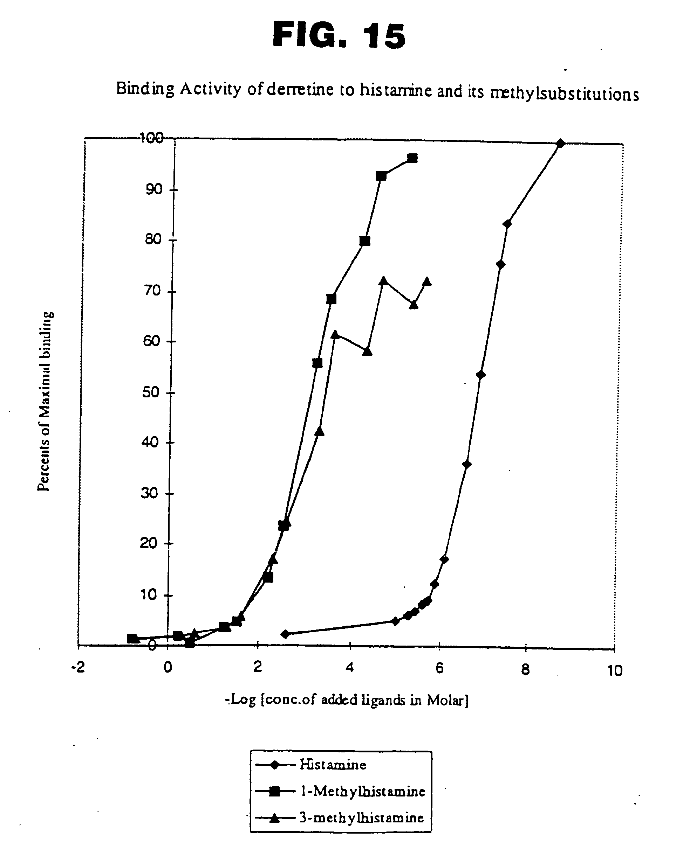

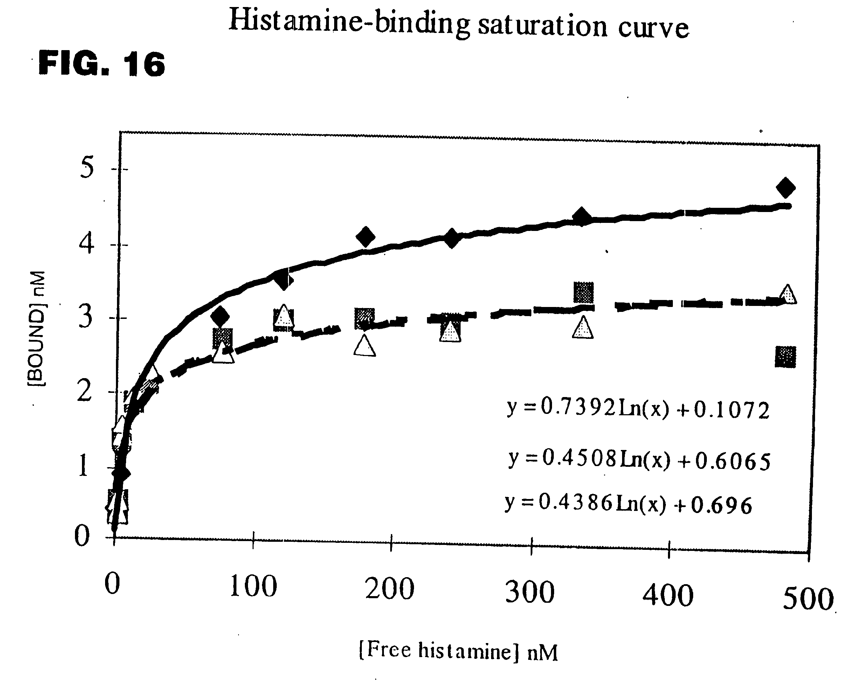

From the plotted curved line, two asymtotic straight lines were drawn according to Feldman (1972) consistent with two histamine binding sites of approximate Kd 6 x 10⁻⁸ M and 2 x 10⁻⁶ M. Comparison of the ability of histamine and its methylderivatives to displace radioactive histamine indicated that the binding by D.RET6 was specific for histamine (Figure 15). Surprisingly, the saturation curves and corresponding Scatchard plots for histamine binding in the presence of serotonin revealed a marked synergistic effect (Figures 15 and 16). At a final concentration of 2.38µM serotonin, the Kd for the two binding sites for histamine was 1.1 x 10⁻⁹ M and 1 x 10⁻⁶ M, and with 23.8µM serotonin, 1.3 x 10⁻⁹ M and 1 x 10⁻⁶ M, respectively.

Thus, in the presence of serotonin, the binding affinity of D.RET6 for its ligand, histamine, was found to increase sixty-fold. Recently, it has been reported that external stimuli (including serotonin) regulate mammalian H1 receptor activity, provoking increased ligand affinity (Bloemers, S. M., Verheule, S., Peppelenbosch, M. P., Smit, M. J., Tertoolen, L. G.J., and De Laat, S. (1998) *The Journal of Biological Chemistry* 273(4), 2249-2255). Although the molecular details remain unclear, a conformational change in the H1 receptor, induced by serotonin, has been proposed to explain the increase in affinity for histamine (Bloemers, 1998). It thus seems possible that the synergistic effect of serotonin on the tick histamine-binding protein has evolved to counteract serotonin-induced enhanced binding affinity of H1 receptors. Such a mechanism might therefore enable the tick protein to outcompete the host's serotonin-sensitized histamine receptors in the feeding site. As adult *D. reticulatus* feed on a variety of domestic and wild mammals, including dog, horse, cattle, sheep, deer, fox, hare and hedgehog, the synergistic effect of serotonin may provide flexibility in the performance of the tick's histamine-binding protein under a range of host-specific haemostatic responses.

This finding has important implications for the design of molecules with histamine binding activity and gives important insights into the mechanism of action of these tick proteins, along with molecules designed to mimic their action. For example, in order to alter the affinity of these molecules for histamine, serotonin may be delivered simultaneously in an appropriate amount.

### Example 4: Crystallisation of proteins

Purified FS-HBP2 was dialysed against 10 mM histamine in water (the pH of the histamine solution was adjusted to 6.8 using NaOH), and concentrated using Centricon 10 centrifugation units (Amicon) to a final protein concentration of 20µg/µl. A hanging drop of 3µl of the protein/histamine solution combined with 2µl of mother liquor was allowed to equilibrate at room temperature with 1ml of mother liquor [0.1 M MES buffer (pH 6.5), containing 0.01 M cobalt chloride hexahydrate and 1.8 M ammonium sulphate (Hampton Research)].

Native data to 2.24Å resolution and derivative data to 3 Å were collected at room temperature using in-house Mar-research imaging plate detectors attached to Rigaku rotating anode generators. Frozen native and semi-apo data were collected (to 1 .24 Å and 1 .35 Å resolution respectively) at the Brookhaven National Laboratory synchrotron using a Brandeis single module CCD detector (see Table 1). The semi-apo crystal was prepared by immersion in several changes of histamine-free mother liquor during the week prior to data collection. Mother liquor containing 30% glycerol was used as a freezing solution. Crystals were kept at 100K using a cryostream (Oxford Cryosystems). Freezing resulted in a distinct change in unit cell dimensions, with axes b and c smaller and axis a slightly larger than at room temperature. Diffraction data were processed with DENZO and SCALEPACK (Otwinowski and Minor, 1997).

### Phasing and model building

The structure was solved by MIR using cis-platinum and trimethyllead-acetate (Holden and Rayment, 1991) derivatives (Table 1). Initially, 10-15° of data were collected for each new derivative. Data collection was continued only for crystals that showed appreciable isomorphous differences, otherwise they were transferred back to the mother liquor for the next soaking experiment.

Difference Patterson maps allowed two binding sites to be located, by inspection, for each heavy metal. Further sites and the common origin for the two derivatives were found by difference Fourier techniques. Trimethyllead-acetate introduces some degree of non-isomorphism (see Table 1). Data reduction, scaling and calculation of isomorphous differences were carried out with the in-house programs 3DSCALE and DIFFER (Stuart *et al.,* 1979). MIR-phases were calculated with MLPHARE (Otwinowski, 1991), and improved by solvent flattening with the program GAP (Grimes and Stuart, unpublished).

The initial maps revealed the presence of two molecules in the asymmetric unit. The noncrystallographic symmetry operator relating these molecules was determined and refined with GAP starting from the coordinates of the heavy metal binding sites and chosen marker positions from the electron density map. Non-crystallographic symmetry averaging resulted in an electron density map of high quality, which could be readily interpreted. Electron density map interpretation used FRODO (Jones, 1985) and 0 (Jones *et al.,* 1991).

**TABLE 1**

| | Unit Cell (a,b,c) | Resolution (A) | Total # reflections | # Unique reflections | Completeness (%) | Rmerge | % iso-morphous difference | # sites | Phasing power |
|---|---|---|---|---|---|---|---|---|---|
| Native 1 | 77.0,77.8,80.1 | 20-2.27 | 127,028 | 22,222 | 94.8 | 5.2 | - | - | - |
| Cis Pt | 76.9,77.8,80.1 | 20-3 | 35,045 | 9.898 | 97.6 | 8.0 | 20.5 | 6 | 1.6/1.3 |
| TML | 76.2.78.9.80.1 | 20-3 | 41,086 | 9,938 | 97.1 | 7.7 | 32.6 | 2 | 1.0/0.8 |

(a) Structure determination and refinement

| | Native2 | Apo |
|---|---|---|
| Unit cell (a,b,c) | 77.5, 74.8, 78.6 | 77.5, 74.4, 77.9 |
| Resolution range (Å) | 20.0-1.24 | 20-1.35 |
| No.of reflections | 108,735 | 76,301 |
| R-factor | 18.4% | 18.7% |
| No.of protein atoms | 2744 | 2744 |
| No.of water molecules | 529 | 537 |
| RMS bond length deviation | 0.013Å | 0.014Å |
| RMS bond angle deviation | 1.5° | 1.6° |
| Mean B-factor (Protein:A&B) | 11Å² | 20² |
| Mean B-factor (ligand, site *H*) | 5Å² | 17Å² |
| Mean B-factor (ligand, site *L*) | 5Å² | § |
| Mean B-factor (water) | 26Å² | 36Å² |

(b) Refinement Statistics

### Refinement and analysis

The initial map immediately revealed that FS-HBP2 had lipocalin topology. The coordinates for retinoic acid binding protein (IRBP) were used as an initial framework for model building. The initial model was refined against the room temperature dataset (Nativel, Table 1) using XPLOR (Brtinger, 1992) (rigid body, positional and B-factor refinement). The resulting model was refined against the higher resolution (Native2) data. Rigid body refinement yielded improved NCS operators which allowed positional B-factor and positional refinement to 1.24Å resolution. Further refinement included overall anisotropic B-factor refinement and simulated annealing. Water and histamine molecules were then included along with a bulk solvent correction. During the course of the refinement stereochemical restraints were modified so that eventually electrostatic and van der Waals terms were omitted from the target function.

PROCHECK (Laskowski *et al.,* 1993) was used for structure validation. Secondary structure assignments used DSSP (Kabsch and Sander, 1983). Structural superpositions used SHP (Stuart *et al.,* 1979). Atomic coordinates used for comparisons were obtained from Protein Data Bank, Brookhaven National Laboratory.

### Structure Determination of rFS-HBP2

The FS-HBP2-histamine complex crystallizes in an orthorhombic space group (P2₁2₁2₁) with 2 molecules per asymmetric unit. The unit cell dimensions vary, especially upon freezing (Table I). The structure was solved by the use of two heavy atom derivatives and refined using XPLOR (Brunger, 1992) to yield a final model with reasonable stereochemistry (RMS bond deviation 0.013 Å) and R-factor of 18.4%, for all data in the 20-1.24 Å resolution range (Table 1). 91.1 % of residues are within the most favoured region of the Ramachandran plot, no residues are in the disallowed region. The two crystallographically distinct molecules (A and B) are very similar (rms deviation for C~ atoms 0.6 Å). Significant differences are in loops at crystal contact points (Figure 20) (the sidechain conformation of residue Val21 also differs between the A and B molecules, but this is unlikely to affect biological function). Both molecules contain two histamines, bound at sites denoted H and L. The error in the co-ordinates for the majority of nonhydrogen atoms is less than 0.2 Å (Luzzatti, 1952).

The *A* and *B* molecules in the crystal have one extensive contact, main chain hydrogen bonds link a short strand following the α3-helix of molecule *A* (residues *A*143 to *A*146) to the end of strand B of molecule *B* (residues *B*54 to *B*57) (Figure 20a,c). Other hydrogen bonds link *A*150 ND2 to *B*25 0 and *A*167 OG to *B*59 OD 1. These molecules are not related by a two-fold axis. Several residues on the surface of the protein possess multiple conformations in the crystal, however these do not have a role in the structural and functional interactions discussed below. In line with the excellent diffraction, the crystallographic B-factors are rather low (Table 1), indicating that the molecule has a core of considerable rigidity. The refined model for molecule *A* consists of residues *A*1 - *A* 171 of the native protein along with the engineered carboxyl-terminal Ile-(His)6 tag. Although the course of the polypeptide chain could be traced in this region, the positions of three of the His side chains was unclear and so they were modelled as Ala. No density was observed for the (His)6 tag in molecule *B* (residue *B*1 was also omitted due to disorder).

### Overall Structure

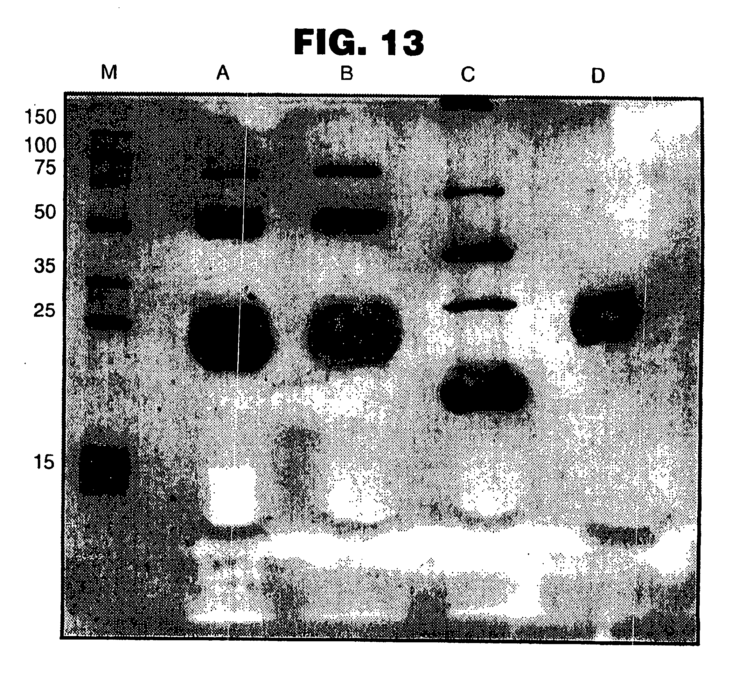

The bulk of the molecule (approximate dimensions 33 Å x 38 Å x 45 Å) is an eight stranded anti-parallel β-barrel, whose topology places FS-HBP2 within the lipocalin superfamily (Figure 13 & Flower, 1996). Lipocalins are, typically, extracellular proteins which transport small hydrophobic ligands with varying degrees of specificity. The HBP β-barrel houses two histamine molecules and the protein is completed by partly helical amino-terminal and carboxyterminal extensions to the β-barrel (Figure 13a). From the high level of sequence similarity, we expect the overall structures of all three Ra-HBPs to be very similar (RMS deviation between Cα atoms ~1 Å).

### Unique Features

Several features of functional significance set FS-HBP2 apart from other lipocalins.

The unusually extended amino terminus of FS-HBP2 initially runs along the barrel, forming hydrogen bond interactions with β-strands F and G before forming the α1 and α2 helix. The substantial α2 helix is positioned over the 'mouth' of the barrel, interacting with residues on the rim to form a significant barrier between the binding pockets and the exterior (Figure 20a,b). One side of the helix is held in place by hydrogen bonds to the loop joining strands B and C. The .other side of the helix hydrogen bonds to strands F and G (the F-G loop is particularly prominent in HBPs, in line with a functional role).

As with other lipocalins there is an α3-helix, which abuts the mid-point of the barrel. In FS-HBP2, the helix is pinned to the barrel at its extremities by two disulphide bridges (Cys119 to Cys148 and Cys48 to Cys168).

Three structurally conserved regions (SCRs), lying in close proximity in the structure, have been identified as determinants of the lipocalin fold (Flower, 1996). SCRI includes a 3/10 helix followed by a Gly-X-Trp sequence in strand A. In FS-HBP2 an α-helix replaces the 3/10 helix and Asn-Val-Tyr (residues 27-29) replaces the signature sequence. In several lipocalins SCR2 contains a Thr-Asp-Tyr motif. Whilst FS-HBP2 does not have this signature sequence there are conformational similarities in this region (such as a characteristic kink in strand F). The final conserved region, between strand H and α3, contains an Arg or Lys residue, nearby the conserved Trp residue of SCRI. In HBP2 this loop contains the tripeptide Thr-Asp-Tyr (139-141), which is usually present in SCR2. Surprisingly, HBP2 contains several other elements of sequence that are also reminiscent of misplaced lipocalin fragments (although the Glu-Lys-Val-Thr-Ala sequence in strand D is a perfect match with the equivalent portion of epididymal retinoic acid binding protein; Newcomer, 1993).

Perhaps the most striking unique feature of the HBP structure is the presence of the two ligand binding sites within the barrel core. One of the histamine molecules binds across the width of the binding pocket, requiring strands G and H to be stripped away from the rest of the sheet, distorting the canonical hydrogen bonding pattern.

### Two Binding Sites which Differ in Affinity for Histamine

To dissect out the relative affinities of the two histamine binding sites, and probe the conformational implications of histamine binding, we attempted to remove histamine from preformed crystals. A rigorous regime of immersion of crystals in mother liquor (minus histamine) was followed by data collection at 100K. The diffraction data revealed that histamine was lost from one of the binding sites in both crystallographically independent molecules whilst the second site remained fully occupied. We infer from this that, in the crystal, one site has a far slower off-rate than the other. The similar behaviour of two molecules in different environments within the crystal strongly suggests that one site, termed H, has a higher intrinsic affinity than the other, termed L. The ligand-protein interactions observed support this assignment.

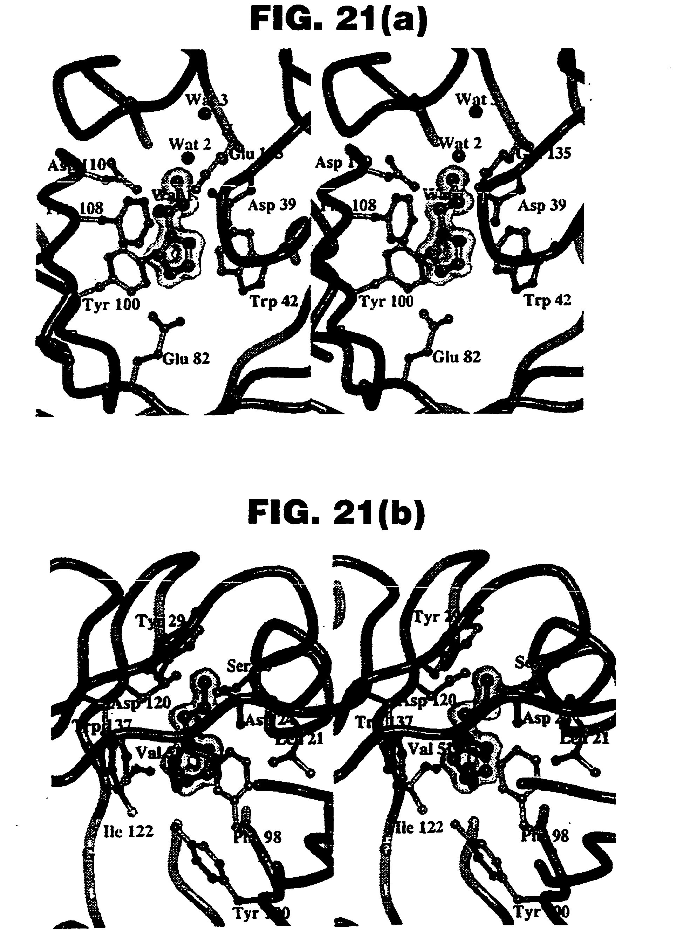

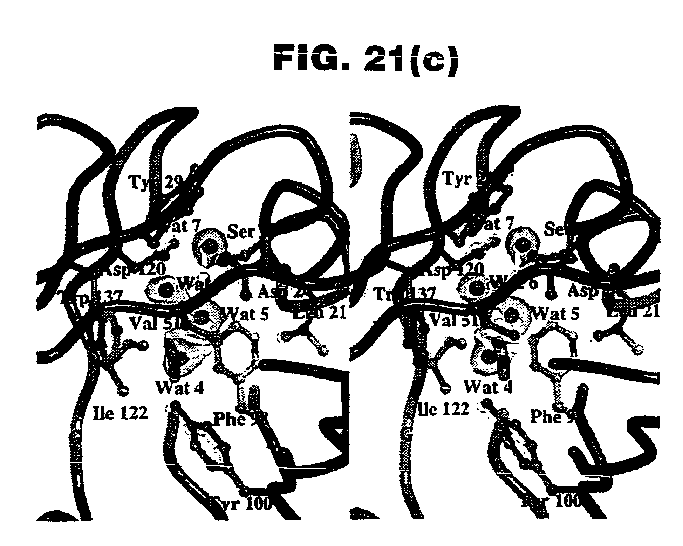

The binding sites are located, off-centre, at opposing ends of the barrel (Figure 20a). Unusually for a lipocalin (but in accord with the hydrophilic nature of the ligand) both binding pockets sequester a number of charged residues in the interior of the barrel. These residues are critical components of the histamine binding Sites (Figure 21 a,b). The RMS difference in the length of the stabilizing hydrogen bonds between the A and B molecules is 0.08 Å; we therefore make no distinction between the two molecules in this respect. The side-chain of residue Tyr100 and a bridging water molecule form a wall separating the H and L sites. Nevertheless a hydrogen bond network links the two sites, the components of which are almost completely conserved between different HBPs. In the H site the histamine ligand lies perpendicular to the long axis of the barrel, leading to distortions in the structure of HBP, compared to other lipocalins. The aromatic side chains of Trp42 and PhelO8 are arranged, parallel but slightly off-centre, to form strong Π-Π stacking interactions (McGaughey *et al.,* 1998) with the imidazole of the histamine molecule. The phenolic ring of Tyr100 is perpendicular to the plane of the imidazole ring of the histamine, contributing further Π-Π interactions. There is an extensive network of hydrogen bonds between the nitrogen atoms of the histamine and the carboxylates of Glu (82 and 135) and Asp (39 and 110) residues, both directly and via bridging water molecules. From its environment, it is expected that the H-site histamine is bound in the di-cationic form.

In the L site the histamine molecule is parallel to the barrel with the imidazole moiety pointing toward the barrel centre. The types of interactions at the L site are similar to the H site but rather less favourable. The imidazole ring of the histamine has base stacking interactions with the phenolic ring of the Phe98 side chain, which are almost perpendicular. There are further van der Waals interactions with the phenolic ring of Tyr29 while Ser20, Asp24 OEI, Tyr100 OH and Aspl2O OE2 form hydrogen bonds with the histamine nitrogen atoms.

Tyr100 therefore has a role in binding both of the histamine molecules, albeit using different modes of interaction. No ordered water molecules are present in the L site and volume calculations suggest that the histamine is slightly less tightly packed in this site. B-factor analysis confirms, however that both histamine ligands are rigidly bound (Table I). The poorer fit of histamine in the L site, raises the question whether a compound other than histamine could be the natural ligand for this pocket.

### Structure of FS-HBP2 with One Histamine Bound

The structure of FS-HBP2 without the histamine in the L site was determined in both molecule A and B, although in molecule B a remnant of histamine (approximately 10% occupancy) is seen. The model for this 'semi-apo' structure has an R-factor of 18.7% (Table 1). The structural details are essentially the same for molecule A and B. Four water molecules have replaced the histamine in the L site, one of which takes the position of the histamine Cβ atom (Figure 21c), whilst the others cushion destabilization by occupying the equivalent positions to the nitrogen atoms of the histamine. Removal of the histamine from the L site causes only slight of (of the order of a few tenths of an Angstrom; the overall RMS change in Cot positions is only 0.1 Å) and side chain conformations remain essentially unchanged, (Figure 21c). The histamine remains bound at full occupancy at the H site, consistent with a much higher affinity.

### Implications of Sequence Variation within the HBP Family

Sequence variation amongst the three HBPs occurs even for residues directly involved in histamine binding, especially those contributing to the L pocket. Overall FS-HBP2 resembles FS-HBP1 more closely than MS-HBP1, however The L-pocket of FS-HBP2 is in fact more similar to that of MS-HBP1 than FS-HBP1, despite the fact that, overall, FS-HBP2 resembles FS-HBP1 most strongly. In FS-HBP1, histidine and tyrosine residues replace Ser2O and Asp12O, respectively. These changes must substantially modify both the shape and charge characteristics of the binding pocket. In contrast to the numerous changes in the L site, only the substitution of Phe 108 for a Leu impinges upon the H site of FS-HBP1. The Kds for FS-HBP2 and MS-HBP1 are similar, and about 10 times lower than that for FS-HBP1, suggesting that modifications at the L site modulate the observed binding affinities. Modelling the FS-HBP1 structure indicates that the loss of aromatic residues may open up an internal cavity, presumably changing the specificity.

MS-HBP3, the male specific protein, has more substantial changes compared to FS-HBP2, including insertions and deletions in the loop regions. MS-HBP1 contains an additional Cys (150), which is likely to be on the surface of the molecule and may be responsible for the formation of the covalent dimers discussed above. The putative glycosylation site, Asn61 is also likely to be exposed to the solvent (Figure 13).

**Table 2. Average Hst - Peptide Distances**

| Interaction | Distance Å |
|---|---|
| Hst Tail -- Glu Acid C | 3.66 |
| Imidazole ring - Glu Acid C | 3.53 |
| Imidazole ring - Phe Centroid | 4.91 |
| Imidazole ring - Trp Centroid | 4.94 |

### Atom Numbering System.

**Table 3. Strong Binding Sites**

| Hst Atom | Residue | Atom | A-Chain Å | B-Chain Å |
|---|---|---|---|---|
| 1 | Tyr 36 | OH | 3.32 | 3.4 |
| 1 | Asp 110 | Acid C | 3.67 | 3.66 |
| 1 | Glu 135 | " | 3.88 | 3.84 |
| 2 | Glu 82 | " | 3.33 | 3.37 |
| 3 | Asp 39 | " | 3.44 | 3.35 |
| 3 | Trp 42 | Centroid of Ring | 3.7 | 3.97 |
| 3 | Phe 108 | " | 4.1 | 4.21 |

**Table 4. Hst Weak Binding Sites**

| Hst Atom | Residue | Atom | A-Chain Å | B-Chain Å |
|---|---|---|---|---|
| 1 | Ser 20 | C=O | 2.85 | 2.83 |
| 1 | Ser 20 | OH | 3.07 | 3.13 |
| 1 | Asp 24 | Acid C | 2.88 | 3.12 |
| 1 | Tyr 29 | Centroid | 3.23 | 3.21 |
| 1 | Asp 120 | O | 3.64 | 2.71 |
| 2 | Asp 24 | O | 2.64 | 2.66 |
| 3 | Tyr 100 | OH | 2.80 | 2.78 |

### REFERENCES

Blake, M.S., Johnston, K.H., Russell-Jones, G.J., Gotschlich, E.C. (1984) A rapid, sensitive method for detection of alkaline-phosphatase-conjugated anti-antibody on Western blots. *Anal. Biochem.* **136**, 175-179.
D.M. Glover, ed., *DNA cloning: a practical approach, Volume II: Expression systems,* (IRL Press, 1995).
D.M. Glover, ed., *DNA cloning: a practical approach, Volume IV: Mammalian systems,* (IRL Press, 1995).
Falus A. (1994) Histamine and Inflammation, R.G. Landes Company, Austin, 139.
Gantz I., DelValle J., Wang L-d., Tashiro T., Munzert T., Guo Y-J., Konda Y. and Yamada T. (1992) *J. Biol. Chem.* **267**, 20840-20843.
Goode, B.L. and Feinstein, S.C. (1992) "Speedprep" purification of template for double-stranded DNA sequencing. *Bio Techniques* **12,** 374-375.
Hogan J.C. (1997) *Nature Biotech.* **15,** 328-330.
Holden H.M. and Rayment I. (1991) *Archives of Biochemistry and Biophysics* **291,** 187-194.
Janknecht R., de Martynoff G., Lou L., Hipskind R.A., Nordheim A. and Stunnenberg H.G. (1991) Rapid and efficient purification of native histidine-tagged protein expressed by recombinant vaccinia virus. *PNAS USA* **88,** 8972-8976.
Jones L.D., Davies C.R., Steele G.M. and Nuttall P.A. (1988). The rearing and maintenance of Ixodid and Argasid ticks in the laboratory. *Animal Technology* **39,** 99-106.
Kitts P.A. and Possee R.D. (1993) A method for producing recombinant baculovirus expression vectors at high frequency. *BioTechniques* **14**, 810-817.
Kyhse-Anderson J. (1984) Electroblotting of multiple gels: a simple apparatus without buffer tank for rapid transfer of proteins from polyacrylamide to nitrocellulose. *J. Biochem. Biophys. Methods* **10,** 203-209.
Laemmli V.K. (1970) Cleavage of structural proteins during the assembly of the head of bacteriophage T4. *Nature* **277.** 680-685.
Livingstone C. and Jones I. (1989) Baculovirus expression vectors with single strand capability. *Nucleic Acids Res.* **17**, 2366.
Maley F., Trimble R.B., Tarentino A.L. and Plummer T.H. Jr. (1989) Characterization of glycoproteins and their associated oligosaccharides through the use of endoglycosidases. *Anal. Biochem.* **180**, 195-204.
Matsudaira P. (1987). Sequence from picomole quantities of proteins electroblotted onto polyvinylidene difluoride membranes. *J.Biol. Chem.* **262,** 10035-10038.
Mierendorf R.C. and Pfeffer D. (1987) Direct sequencing of denatured plasmid DNA. *Methods Enzymol.* **152,** 556-562.
*O'Reilly et al.,* (1994) *Baculovirus expression vectors - a laboratory manual,* Oxford University Press.
Program Manual for the Wisconsin Package, version 8 (1994) Genetics Computer Group, 575 Science Drive, Madison, Wisconsin, USA 53711.
Protein Expression in Current Protocols in Molecular Biology (Chapter 16). Editors : F.M. Ausubel, R. Brent, R.E. Kingson, D.D. Moore, J.G. Seidman, J.A. Smith, K. Struhl. Published by John Wiley and Sons, Inc.
Rees A.R. *et al.,* eds, *Protein Engineering: A practical approach,* IRL Press 1993.
Ribeiro J.M. and Walker F.A. (1994) High-affinity histamine-binding and antihistaminic activity of the salivary nitric oxide-carrying heme protein (nitrophorin) of Rhodnius prolixus. *J. Exp. Med.* **180**, 2251-2257.
Sambrook J., Fritsch E.F. and Maniatis T. (1989) Molecular Cloning: A Laboratory Manual. Cold Spring Harbour Laboratory.
Sanger F. and Coulson A.R. (1975). A rapid method for determining sequences in DNA by primed synthesis with DNA polymerase. *J.Mol. Biol.* **94,** 441-448.
Schägger, H. and von Jagow, G. (1987) Tricine-sodium dodecyl sulfate-polyacrylamide gel electrophoresis for the separation of proteins in the range from 1 to 100kDa. *Anal. Biochem.* **166**, 368-379.
Short J.M., Fernandez J.M., Sorge J.A. and Juse W.D. (1988) λZAP: a bacteriophage λ expression vector with *in vivo* excision properties. *Nucl. Acids Res.* **16**, 7583-7600.
Towbin, H., Staeholin, T. and Gordon, J. (1979) Electrophoretic transfer of proteins from polyacrylamid gels to nitrocellulose sheets : Procedure and some applications. *Proc. Natl. Acad. Sci. USA* **76,** 4350-4354.
Wang H. and Nuttal P.A. (1995). Immunoglobulin G binding proteins in male *Rhipicephalus appendiculatus* ticks. *Parasite Immunology* **17**, 517-524.
Warlow R.S. and Bernard C.C.A. (1987). Solubilization and characterization of moderate and high affinity histamine binding sites on human blood mononuclear cells. *Molec. Immun.* **24**, 27-37.
Watson *et al.,* Recombinant DNA (2nd edition), Scientific American Books.

## Claims

1. Use of a serotonin binding compound capable of binding to serotonin with a dissociation constant of less than 10⁻⁷M and which has a binding site comprising amino acid residues phenylalanine, isoleucine or leucine at position I, tryptophan at position II and aspartate or glutamate at positions III and IV wherein residues I to IV are positioned substantially the same as residues 108, 42, 39 and 82 respectively in either of Figures 1 or 2, or residues 107, 41, 38 and 78 in Figure 3 or residues 122, 54, 50 and 95 in Figure 4, wherein the numbering of the amino acid residues refers to the sequence of the mature protein that lacks the leader sequence, in the manufacture of a medicament for the treatment or prevention of a disease condition in which serotonin is implicated, and wherein said disease condition is abnormal blood pressure, migraine, a psychological disorder, respiratory disease or coronary heart disease.

2. Use of serotonin binding compound capable of binding to serotonin with a dissociation constant of less than 10⁻⁷M and which has a binding site comprising amino acid residues phenylalanine or isoleucine at residue I, tryptophan at residue II and aspartate or glutamate at residues III and IV wherein residues I to IV are petitioned substantially the same as residues 98, 137, 24 and 120 respectively in either of Figures 1 or 2, or residues 95, 138, 23 and 120 in Figure 3 or residues 112, 149, 35 and 135 in Figure 4, wherein the numbering of the amino acid residues refers to the sequence of the mature protein that lacks the leader sequence, in the manufacture of a medicament for the treatment or prevention of a disease condition in which serotonin is implicated, and wherein said disease condition is abnormal blood pressure, migraine, a psychological disorder, respiratory disease or coronary heart disease.

3. Use of a serotonin binding compound capable of binding to serotonin with a dissociation constant of less than 10⁻⁷M and which has two binding sites, the first binding site comprising amino acid residues phenylalanine, isoleucine or leucine at position I, tryptophan at position II and aspartate or glutamate at positions III and IV wherein residues I to IV are positioned substantially the same as residues 108, 42, 39 and 82 respectively in either of Figures 1 or 2, or residues 107, 41, 38 and 78 in Figure 3 or residues 122, 54, 50 and 95 in Figure 4, and the second binding site comprising amino acid residues phenylalanine or isoleucine at residue I, tryptophan at residue II and aspartate or glutamate at residues III and IV wherein residues I to IV are positioned substantially the same as residues 98, 137, 24 and 120 respectively in either of Figures 1 or 2, or residues 95, 138, 23 and 120 in Figure 3 or residues 112, 149, 35 and 135 in Figure 4, wherein the numbering of the amino acid residues refers to the sequence of the mature protein that lacks the leader sequence, in the manufacture of a medicament for the treatment or prevention of a disease condition in which serotonin is implicated, and wherein said disease condition is abnormal blood pressure, migraine, a psychological disorder, respiratory disease or coronary heart disease.

4. Use according to claim 1 or claim 3, wherein said serotonin binding compound additionally comprises at residue V, a tyrosine residue, wherein residue V is positioned substantially the same as residue 100 in the sequence of either of Figures 1 or 2, residue 97 in Figure 3 or residue 114 in Figure 4, wherein the numbering of the amino acid residues refers to the sequence of the mature protein that lacks the leader sequence.

5. Use according to claim 2 or claim 3, wherein said serotonin binding compound additionally comprises at residue V, a tyrosine residue, wherein residue V is positioned substantially the same as residue 29 in the protein sequence of either of Figures 1 or 2, residue 28 in Figure 3 or residue 40 in Figure 4, wherein the numbering of the amino acid residues refers to the sequence of the mature protein that lacks the leader sequence.

6. Use according to any preceding claim wherein said compound is stabilised by either or both of the disulphide bridges formed between cysteines 48 and 169 and cysteines 148 and 119 in the protein sequence of either of Figures 1 or 2, cysteines 47 and 175 and cysteines 151 and 119 of Figure 3 or cysteines 162 and 134 of Figure 4, wherein the numbering of the amino acid residues refers to the sequence of the mature protein that lacks the leader sequence.

7. Use according to any one of the preceding claims, wherein said compound comprises a peptide, or a fragment of any one of the proteins whose amino acid sequences are presented in Figures 1-4, wherein said peptide or fragment is capable of binding to serotoin with a dissociation constant of less than 10⁻⁷M and has a binding site as recited in any one of claims 1-6.

8. Use according to claim 7, wherein said compound comprises a cyclic peptide.

9. Use according to claim 8, wherein said cyclic peptide comprises the sequence Ala-Glu-Ala-Phe-Ala-Glu-Ala-Trp.

10. Use according to any one of claims 1 to 9, wherein said serotonin binding compound is a synthetic compound.

11. Use according to any one of claims 1 to 10, wherein said serotonin binding compound is produced by recombinant DNA technology.

12. Use according to any one of claims 1 to 11, wherein said serotonin binding compound has an effector or reporter molecule attached thereto.

13. Use according to any one of the preceding claims, wherein said serotonin binding compound is derived from ticks.

14. Use according to claim 13, wherein said serotonin binding compound is derived from Ixodid ticks.

15. Use according to claim 14, wherein said serotonin binding compound is derived from *Rhipicephalus appendiculatus, D. reticulatus, Amblyomma variegatum, Boophilus microplus or Ixodes hexagonus.*

16. Use according to any one of claims 1 to 15, wherein said serotonin binding compound is genetically or chemically fused to one or more peptides or polypeptides.

17. Use according to any one of claims 1 to 16, wherein said serotonin binding compound or protein of any one of the preceding claims is attached to a label or toxin.

18. Use according to any one of claims 1 to 17, wherein said serotonin binding compound or protein is bound to a support, such as a resin.

19. A therapeutic or diagnostic composition comprising a serotonin binding compound as recited in any one of the preceding claims additionally comprising serotonin.

20. A therapeutic or diagnostic composition according to claim 19 additionally comprising a cysteinyl leukotriene, platelet activating factor, or a thromboxane.

## Patentansprüche

1. Verwendung einer Serotonin-bindenden Verbindung, befähigt zum Binden an Serotonin, mit einer Dissoziationskonstanten von weniger als 10⁻⁷M, und welche eine Bindungsstelle aufweist, umfassend Aminosäurreste, Phenylalanin, Isoleucin oder Leucin an Position I, Tryptophan an Position II und Aspartat oder Glutamat an Positionen III und IV, wobei die Reste I bis IV im wesentlichen die gleiche Position aufweisen wie die Reste 108, 42, 39 bzw. 82 in einer der Figuren 1 oder 2, oder die Reste 107, 41, 38 und 78 in Figur 3, oder die Reste 122, 54, 50 und 95 in Figur 4, wobei sich die Numerierung der Aminosäurereste auf die Sequenz des reifen Proteins bezieht, welchen die Leadersequenz fehlt, zur Herstellung eines Medikaments für die Behandlung oder Vorbeugung eines Krankheitszustands, in welchen Serotonin verwickelt ist, und wobei der Krankheitszustand anormaler Blutdruck, Migräne, eine psychologische Störung, eine Atmungserkrankung oder eine koronare Herzerkrankung ist.

2. Verwendung einer Serotonin-bindenden Verbindung, befähigt zum Binden an Serotonin, mit einer Dissoziationskonstanten von weniger als 10⁻⁷ M, und welche eine Bindungsstelle aufweist, umfassend die Aminosäurereste Phenylalanin oder Isoleucin bei Rest I, Tryptophan bei Rest II und Aspartat oder Glutamat bei Resten III und IV, wobei die Reste I bis IV im wesentlichen die gleiche Position aufweisen wie die Reste 98, 137, 24 bzw, 120 in einer der Figuren 1 oder 2, oder die Reste 95, 138, 23 und 120 in Figur 3, oder die Reste 112, 149, 35 und 135 in Figur 4, wobei sich die Numerierung der Aminosäurereste auf die Sequenz des reifen Proteins bezieht, welchem die Leadersequenz fehlt, zur Herstellung eines Medikaments für die Behandlung oder Vorbeugung eines Krankheitszustands, in welchen Serotonin verwickelt ist, und wobei der Krankheitszustand anormaler Blutdruck, Migräne, eine psychologische Störung, eine Atmungserkrankung oder eine koronare Herzerkrankung ist.

3. Verwendung einer Serotonin-bindenden Verbindung, befähigt zum Binden an Serotonin mit einer Dissoziationskonstante von weniger als 10⁻⁷ M, und welche zwei Bindungsstellen aufweist, wobei die erste Bindungsstelle die Aminosäurereste Phenylalanin, Isoleucin oder Leucin bei Position I, Tryptophan bei Position II und Aspartat oder Glutamat bei Positionen III oder IV umfaßt, wobei die Reste I bis IV im wesentlichen die gleiche Position aufweisen wie die Reste 108, 42, 39 bzw, 82, in einer der Figuren 1 oder 2, oder die Reste 107, 41, 38 und 78 in Figur 3, oder die Reste 122, 54, 50 und 95 in Figur 4 umfaßt, und die zweite Bindungsstelle die Aminosäurereste Phenylalanin oder Isoleucin bei Rest I, Tryptophan bei Rest II und Aspartat oder Glutamat bei den Resten III und IV umfaßt, wobei die Reste I bis IV im wesentlichen die gleiche Position aufweisen wie die Reste 98, 137, 24 bzw. 120 in einer der Figuren 1 oder 2, oder die Reste 95, 138, 23 und 120 in Figur 3, oder die Reste 112, 149, 35 und 135 in Figur 4, wobei sich die Numerierung der Aminosäurereste auf die Sequenz des reifen Proteins bezieht, welchem die Leadersequenz fehlt, zur Herstellung eines Medikaments für die Behandlung oder Vorbeugung eines Krankheitszustands, in welchen Serotonin verwickelt ist, und wobei der Krankheitszustand anormaler Blutdruck, Migräne, eine psychologische Störung, eine Atmungserkrankung oder eine koronare Herzerkrankung ist.

4. Verwendung nach Anspruch 1 oder 3, wobei die Serotonin-bindende Verbindung zusätzlich einen Tyrosinrest bei Rest V umfaßt, wobei der Rest V im wesentlichen die gleiche Position aufweist wie der Rest 100 in der Sequenz von einer der Figuren 1 oder 2, der Rest 97 in Figur 3, oder der Rest 114 in Figur 4, wobei sich die Numerierung der Aminosäurereste auf die Sequenz des reifen Proteins bezieht, welchem die Leadersequenz fehlt.

5. Verwendung nach Anspruch 2 oder 3, wobei die Serotonin-bindende Verbindung zusätzlich einen Tyrosinrest bei Rest V umfaßt, wobei der Rest V im wesentlichen die gleiche Position aufweist wie der Rest 29 in der Proteinsequenz von einer der Figuren 1 oder 2, der Rest 28 in Figur 3, oder der Rest 40 in Figur 4, wobei sich die Numerierung der Aminosäuresereste auf die Sequenz des reifen Proteins bezieht, welchem die Leadersequenz fehlt.

6. Verwendung nach einem vorangehenden Anspruch, wobei die Verbindung durch eine oder beide der Disulfidbrücken stabilisiert ist, welche zwischen Cysteinen 48 und 169 und Cysteinen 148 und 119 in der Proteinsequenz von einer der Figuren 1 oder 2, Cysteinen 47 und 175 und Cysteinen 151 und 119 von Figur 3, oder Cysteinen 162 und 134 von Figur 4 gebildet sind, wobei sich die Numerierung der Aminosäurereste auf die Sequenz des reifen Proteins bezieht, welchem die Leadersequenz fehlt.

7. Verwendung nach einem der vorstehenden Ansprüche, wobei die Verbindung ein Peptid oder ein Fragment von einem der Proteine, deren Aminosäuresequenzen in Figuren 1-4 dargestellt sind, umfaßt, wobei das Peptid oder Fragment zum Binden an Serotonin mit einer Dissoziationskonstante von weniger als 10⁻⁷ M befähigt ist und eine Bindurigsstelle, wie in einem der Ansprüche 1-6 angeführt ist, aufweist.

8. Verwendung nach Anspruch 7, wobei die Verbindung ein zyklisches Peptid umfaßt.

9. Verwendung nach Anspruch 8, wobei das zyklische Peptid die Sequenz Ala-Glu-Ala-Phe-Ala-Glu-Ala-Trp umfaßt.

10. Verwendung nach einem der Ansprüche 1 bis 9, wobei die Serotonin-bindende Verbindung eine synthetische Verbindung ist.

11. Verwendung nach einem der Ansprüche 1 bis 10, wobei die Serotonin-bindende Verbindung durch rekombinante DNA-Technologie hergestellt ist.

12. Verwendung nach einem der Ansprüche 1 bis 11, wobei die Serotonin-bindende Verbindung ein daran verbundenes Effektor- oder Reportermolekül aufweist.

13. Verwendung nach einem der vorstehenden Ansprüche, wobei die Serotonin-bindende Verbindung von Zecken stammt.

14. Verwendung nach Anspruch 13, wobei die serotonin-bindende Verbindung von Ixodeszecken stammt.

15. Verwendung nach Anspruch 14, wobei die Serotonin-bindende Verbindung von *Rhipicephalus appendiculatus, D. reticulatus, Amblyomma variegatum, Boophilus microplus oder lxodes hexagonus* stammt.

16. Verwendung nach einem der Ansprüche 1 bis 15, wobei die Serotonin-bindende Verbindung genetisch oder chemisch an eines oder mehrere Peptide oder Polypeptide fusioniert ist.

17. Verwendung nach einem der Ansprüche 1 bis 16, wobei die Serotonin-bindende Verbindung oder das Protein von einem der vorstehenden Ansprüche an eine Markierung oder Toxin gebunden ist.

18. Verwendung nach einem der Ansprüche 1 bis 17, wobei die Serotonin-bindende Verbindung oder das Protein an einen Träger, wie ein Harz, gebunden ist.

19. Therapeutische oder diagnostische Zusammensetzung, umfassend eine Serotonin-bindende Verbindung, wie in einem der vorstehenden Ansprüche angeführt, weiche zusätzlich Serotonin umfaßt.

20. Therapeutische oder diagnostische Zusammensetzung nach Anspruch 19, welche zusätzlich ein Cysteinylleukotrien, einen Blutplätichen-aktivierenden Faktor oder ein Thromboxan umfaßt.

## Revendications

1. Utilisation d'un composé de liaison à la sérotonine, capable de se lier à la sérotonine avec une constante de dissociation inférieure à 10⁻⁷ M, et qui possède un site de liaison comprenant les résidus d'acides aminés phénylalanine, isoleucine et leucine sur la position I, tryptophane sur la position II et aspartate ou glutamate sur les positions III et IV, où les résidus I à IV sont positionnés essentiellement de la même manière respectivement que les résidus 108, 42, 39 et 82 sur l'une ou l'autre des Figures 1 ou 2, ou que les résidus 107, 41, 38 et 78 sur la Figure 3, ou que les résidus 122, 54, 50 et 95 sur la Figure 4, où la numérotation des résidus d'acides aminés se rapporte à la séquence de la protéine mature dans laquelle la séquence de tête est manquante, pour préparer un médicament destiné au traitement ou à la prévention d'un état pathologique dans lequel est impliquée la sérotonine, et où ledit état pathologique est une pression sanguine anormale, une migraine, un trouble psychologique, une maladie respiratoire ou une maladie coronarienne.

2. Utilisation d'un composé de liaison à la sérotonine capable de se lier à la sérotonine avec une constante de dissociation inférieure à 10⁻⁷ M et qui possède un site de liaison comprenant les résidus d'acides aminés phénylalanine ou isoleucine sur le résidu I, tryptophane sur le résidu II et aspartate ou glutamate sur les résidus III et IV, où les résidus I à IV sont positionnés essentiellement de la même manière respectivement que les résidus 98, 137, 24 et 120 sur l'une ou l'autre des Figures 1 ou 2, ou que les résidus 95, 138, 23 et 120 sur la Figure 3, ou que les résidus 112, 149, 35 et 135 sur la Figure 4, où la numérotation des résidus d'acides aminés se rapporte à la séquence de la protéine mature dans laquelle la séquence de tête est manquante, pour préparer un médicament destiné au traitement ou à la prévention d'un état pathologique dans lequel est impliquée la sérotonine, et où ledit état pathologique est une pression sanguine anormale, une migraine, un trouble psychologique, une maladie respiratoire ou une maladie coronarienne.

3. Utilisation d'un composé de liaison à la sérotonine capable de se lier à la sérotonine avec une constante de dissociation inférieure à 10⁻⁷ M et qui possède deux sites de liaison, le premier site de liaison comprenant les résidus d'acides aminés phénylalanine, isoleucine ou leucine sur la position I, tryptophane sur la position II et aspartate ou glutamate sur les positions III et IV, où les résidus I à IV sont positionnés essentiellement de la même manière respectivement que les résidus 108, 42, 39 et 82 sur l'une ou l'autre des Figures 1 ou 2, ou que les résidus 107, 41, 38 et 78 sur la Figure 3, ou que les résidus 122, 54, 50 et 95 sur la Figure 4, et le deuxième site de liaison comprenant les résidus d'acides aminés phénylalanine ou isoleucine sur le résidu I, tryptophane sur le résidu II et aspartate ou glutamate sur les résidus III et IV, où les résidus I à IV sont positionnés essentiellement de la même manière respectivement que les résidus 98, 137, 24 et 120 sur l'une ou l'autre des Figures 1 ou 2, ou que les résidus 95, 138, 23 et 120 sur la Figure 3, ou que les résidus 112, 149, 35 et 135 sur la Figure 4, où la numérotation des résidus d'acides aminés se rapporte à la séquence de la protéine mature dans laquelle la séquence de tête est manquante, pour préparer un médicament destiné au traitement ou à la prévention d'un état pathologique dans lequel est impliquée la sérotonine, et où ledit état pathologique est une pression sanguine anormale, une migraine, un trouble psychologique, une maladie respiratoire ou une maladie coronarienne.

4. Utilisation selon la revendication 1 ou 3, pour laquelle ledit composé de liaison à la sérotonine comprend en outre un résidu V, un résidu de tyrosine, où le résidu V est positionné essentiellement de la même manière que le résidu 100 de la séquence de l'une ou l'autre des Figures 1 ou 2, que le résidu 97 sur la Figure 3 ou que le résidu 114 sur la Figure 4, où la numérotation des résidus d'acides aminés se rapporte à la séquence de la protéine mature dans laquelle la séquence de tête est manquante.

5. Utilisation selon la revendication 2 ou 3, pour laquelle ledit composé de liaison à la sérotonine comprend en outre un résidu V, un résidu de tyrosine, où le résidu V est positionné essentiellement de la même manière que le résidu 29 de la séquence protéique de l'une ou l'autre des Figures 1 ou 2, que le résidu 28 sur la Figure 3 ou que le résidu 40 sur la Figure 4, où la numérotation des résidus d'acides aminés se rapporte à la séquence de la protéine mature dans laquelle la séquence de tête est manquante.

6. Utilisation selon l'une quelconque des revendications précédentes, pour laquelle ledit composé est stabilisé par l'un ou l'autre, ou les deux, des ponts disulfure formés entre les cystéines 48 et 169, et entre les cystéine 148 et 119 de la séquence protéique de l'une ou l'autre des Figures 1 ou 2, entre les cystéine 47 et 175 et entre les cystéine 151 et 119 sur la Figure 3 ou entre les cystéines 162 et 134 sur la Figure 4, où la numérotation des résidus d'acides aminés se rapporte à la séquence de la protéine mature dans laquelle la séquence de tête est manquante.

7. Utilisation selon l'une quelconque des revendications précédentes, pour laquelle ledit composé comprend un peptide, ou un fragment de l'une quelconque des protéines dont les séquences d'acides aminés sont présentées sur les Figures 1 à 4, où ledit peptide ou ledit fragment est capable de se lier à la sérotonine avec une constante de dissociation inférieure à 10⁻⁷ M et possède un site de liaison tel que défini dans l'une quelconque des revendications 1 à 6.

8. Utilisation selon la revendication 7, pour laquelle ledit composé comprend un peptide cyclique.

9. Utilisation selon la revendication 8, pour laquelle ledit peptide cyclique comprend la séquence Ala-Glu-Ala-Phe-Ala-Glu-Ala-Trp.

10. Utilisation selon l'une quelconque des revendications 1 à 9, pour laquelle ledit composé de liaison à la sérotonine est un composé synthétique.

11. Utilisation selon l'une quelconque des revendications 1 à 10, pour laquelle ledit composé de liaison à la sérotonine est produit par la technologie de l'ADN recombinant.

12. Utilisation selon l'une quelconque des revendications 1 à 11, pour laquelle ledit composé de liaison à la sérotonine possède une molécule effectrice ou rapporteur, qui y est fixée.

13. Utilisation selon l'une quelconque des revendications précédentes, pour laquelle ledit composé de liaison à la sérotonine est obtenu à partir de tiques.

14. Utilisation selon la revendication 13, pour laquelle ledit composé de liaison à la sérotonine est obtenu à partir de tiques de la famille d'ixodidés.

15. Utilisation selon la revendication 14, pour laquelle ledit composé de liaison à la sérotonine est obtenu à partir de *Rhipicephalus appendiculatus, D. reticulatus, Amblyomma variegatum, Boophilus microplus* ou *Ixodes hexagonus.*

16. Utilisation selon l'une quelconque des revendications 1 à 15, pour laquelle ledit composé de liaison à la sérotonine est génétiquement ou chimiquement fusionné à un ou plusieurs peptides ou polypeptides.

17. Utilisation selon l'une quelconque des revendications 1 à 16, pour laquelle ledit composé de liaison à la sérotonine, ou la protéine selon l'une quelconque des revendications précédentes, est fixé à un marqueur ou à une toxine.

18. Utilisation selon l'une quelconque des revendications 1 à 17, pour laquelle ledit composé de liaison à la sérotonine ou ladite protéine est lié à un support, tel qu'une résine.

19. Composition thérapeutique ou diagnostique comprenant un composé de liaison à la sérotonine tel que défini dans l'une quelconque des revendications précédentes, et comprenant en outre de la sérotonine.

20. Composition thérapeutique ou diagnostique selon la revendication 19, qui comprend en outre un cystéinyl-leucotriène, un facteur d'activation plaquettaire ou un thromboxane.
